(19) Europäisches Patentamt
European Patent Office
Office européen des brevets

(11) Publication number: **0 181 521**
**A1**

(12) **EUROPEAN PATENT APPLICATION**

(21) Application number: 85113203.5

(22) Date of filing: 17.10.85

(51) Int. Cl.[4]: C 07 D 215/56
C 07 D 487/04, A 61 K 31/495
//(C07D487/04, 241:00, 209:00)

(30) Priority: 19.10.84 JP 221070/84
14.11.84 JP 239894/84
30.01.85 JP 17256/85
10.05.85 JP 100056/85
17.07.85 JP 157342/85
19.07.85 JP 160808/85
19.07.85 JP 160809/85
22.07.85 JP 162569/85

(43) Date of publication of application:
21.05.86 Bulletin 86/21

(84) Designated Contracting States:
CH DE FR GB IT LI NL SE

(71) Applicant: OTSUKA PHARMACEUTICAL CO., LTD.
9, Kandatsukasacho 2-chome
Chiyoda-ku Tokyo 101(JP)

(72) Inventor: Tone, Hitoshi
864-20, Omatsu Kawauchi-cho
Tokushima-shi Tokushima-ken(JP)

(72) Inventor: Miyamoto, Hisashi
21-3, Yoshinari Aza Todoroki Ojin-cho
Tokushima-shi Tokushima-ken(JP)

(72) Inventor: Ueda, Hiraki
8-1, Minamisho-machi 3-chome
Tokushima-shi Tokushima-ken(JP)

(72) Inventor: Nakagawa, Kazuyuki
774-1, Omatsu Kawauchi-cho
Tokushima-shi Tokushima-ken(JP)

(74) Representative: von Kreisler, Alek, Dipl.-Chem. et al,
Deichmannhaus am Hauptbahnhof
D-5000 Köln 1(DE)

(54) **Antimicrobial 1-substituted Phenyl-4-oxoquinoline-3-carboxylic acid compounds.**

(57) Novel compounds of the formula:

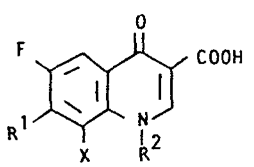

wherein
$R^1$ is a group of the formula:

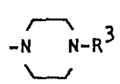

(where $R^3$ is hydrogen atom or an alkyl having 1 to 2 carbon atoms), or a group for the formula:

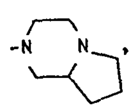

$R^2$ is a group of the formula:

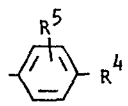

$R^4$ is hydroxy, fluorine atom or an alkanoyloxy having 1 to 6 carbon atoms, $R^5$ is hydrogen or fluorine atom, and X is hydrogen or fluorine atom, provided that when $R^1$ is

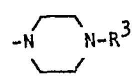

and X is hydrogen atom, $R^2$ is not a group of the formula

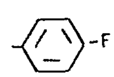 or 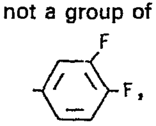

and pharmaceutically acceptable salts thereof, said compounds having excellent antimicrobial activity and hence being useful as an antimicrobial agent, and a pharmaceutical composition comprising as an active ingredient said compounds or a salt thereof in admixture with a pharmaceutically acceptable diluent or carrier.

EP 0 181 521 A1

# ANTIMICROBIAL 1-SUBSTITUTED PHENYL-4-OXOQUINOLINE-3-CARBOXYLIC ACID COMPOUNDS

The present invention relates to novel antimicrobial benzoheterocyclic compounds and salts thereof, more particularly 1-substituted phenyl-4-oxoquinoline-3-carboxylic acid compounds of the formula:

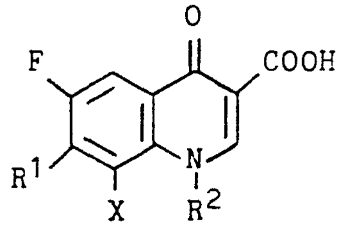

[1]

wherein $R^1$ is a group of the formula: 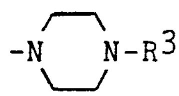 (where $R^3$ is hydrogen atom or an alkyl having 1 to 2 carbon atoms), or a group for the formula: 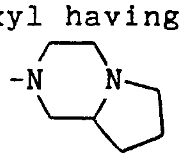 ,

$R^2$ is a group of the formula: 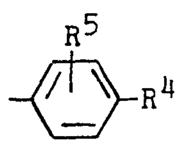

$R^4$ is hydroxy, fluorine atom or an alkanoyloxy having 1 to 6 carbon atoms, $R^5$ is hydrogen atom or fluorine atom, and X is hydrogen atom or fluorine atom, provided that when $R^1$ is 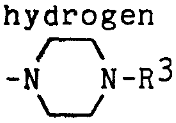 and X is hydrogen atom, $R^2$ is not a group of the formula 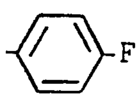 or 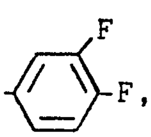 , and pharmaceutically acceptable salts thereof.

The benzoheterocyclic compounds of the formula [1] and salts thereof have excellent antibacterial activities

against various gram positive and gram negative bacteria, and are useful for the treatment of various infectious diseases induced by various bacteria in human, other animals and fish and are also useful as an external antimicrobial or disinfectant agent for medical instruments or the like.

Prior art

The following compounds have been published in Interscience Conference Antimicrobial Agents and Chemotherapy held on October 8-10, 1984 (cf. Abstracts of The 984 ICAAC, page 102, Item 72).

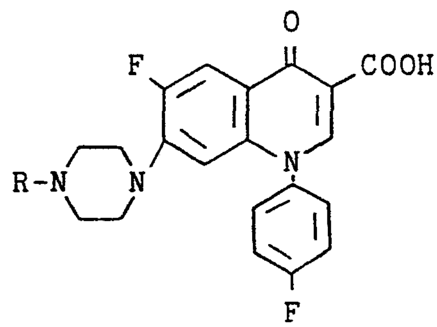

wherein R is hydrogen or methyl.

These known compounds are somewhat similar to the compounds [1] of the present invention in the chemical formula, but the literature does not disclose the specific compounds of the present invention. Besides, the compounds of the present invention show superior antimicrobial activity and absorbability to the above known compounds as is clear from comparative experiments disclosed hereinafter.

Brief Description of the Invention

The object of the present invention is to provide

novel benzoheterocyclic compounds of the formula [1] and salts thereof which have excellent antimicrobial activity and excellent absorbability. Another object of the invention is to provide a pharmaceutical composition containing as an active ingredient a compound of the formula [1] or a pharmaceutically acceptable salt thereof, which is useful for the treatment of various infectious diseases. These and other objects of the invention will be apparent to persons skilled in the art from the following description.

## Detailed Description of the Invention

The novel benzoheterocyclic compounds of the present invention have the formula [1] as mentioned above and include pharmaceutically acceptable salts thereof.

In the formula [1], the term "alkyl having 1 to 2 carbon atoms" for $R^3$ includes methyl and ethyl, preferably methyl. The term "alkanoyloxy having 1 to 6 carbon atoms" for $R^4$ includes formyloxy, acetyloxy, propionyloxy, butyryloxy, isobutyryloxy, pentanoyloxy, hexanoyloxy, etc., preferably an alkanoyloxy having 2 to 4 carbon atoms such as acetyloxy, propionyloxy, butyryloxy and isobutyryloxy, more preferably acetyloxy.

Preferred group $R^1$ is the group of the formula: 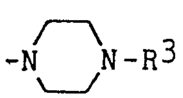 ($R^3$ is an alkyl having 1 to 2 carbon atoms, more preferably methyl) or a group of the formula: 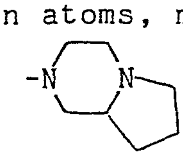 ,

and preferred $R^2$ is a group of the formula:

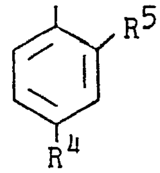

wherein $R^4$ and $R^5$ are as defined above.

The group of the formula: 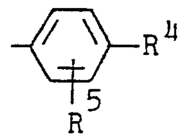 includes specifically 4-fluorophenyl, 2,4-difluorophenyl, 3,4-difluorophenyl, 4-hydroxyphenyl, 4-acetyloxyphenyl, 4-propionyloxyphenyl, 4-hexanoyloxyphenyl, 4-hydroxy-2-fluorophenyl, 4-hydroxy-3-fluoropheny, 4-acetyloxy-2-fluorophenyl, 4-acetyloxy-3-fluorophenyl, and the like. Preferred $R^5$ is hydrogen atom, and preferred $R^4$ is hydroxy and an alkanoyloxy group.

The compounds [1] and their salts of the present invention can be prepared by various processes, for example, by the process as shown in the following reaction schemes-I to -IV.

[Reaction scheme-I]

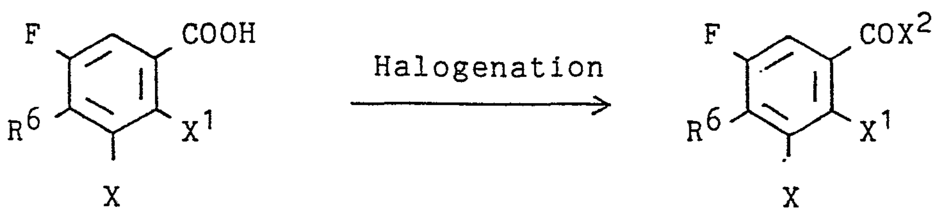


[2] [3]

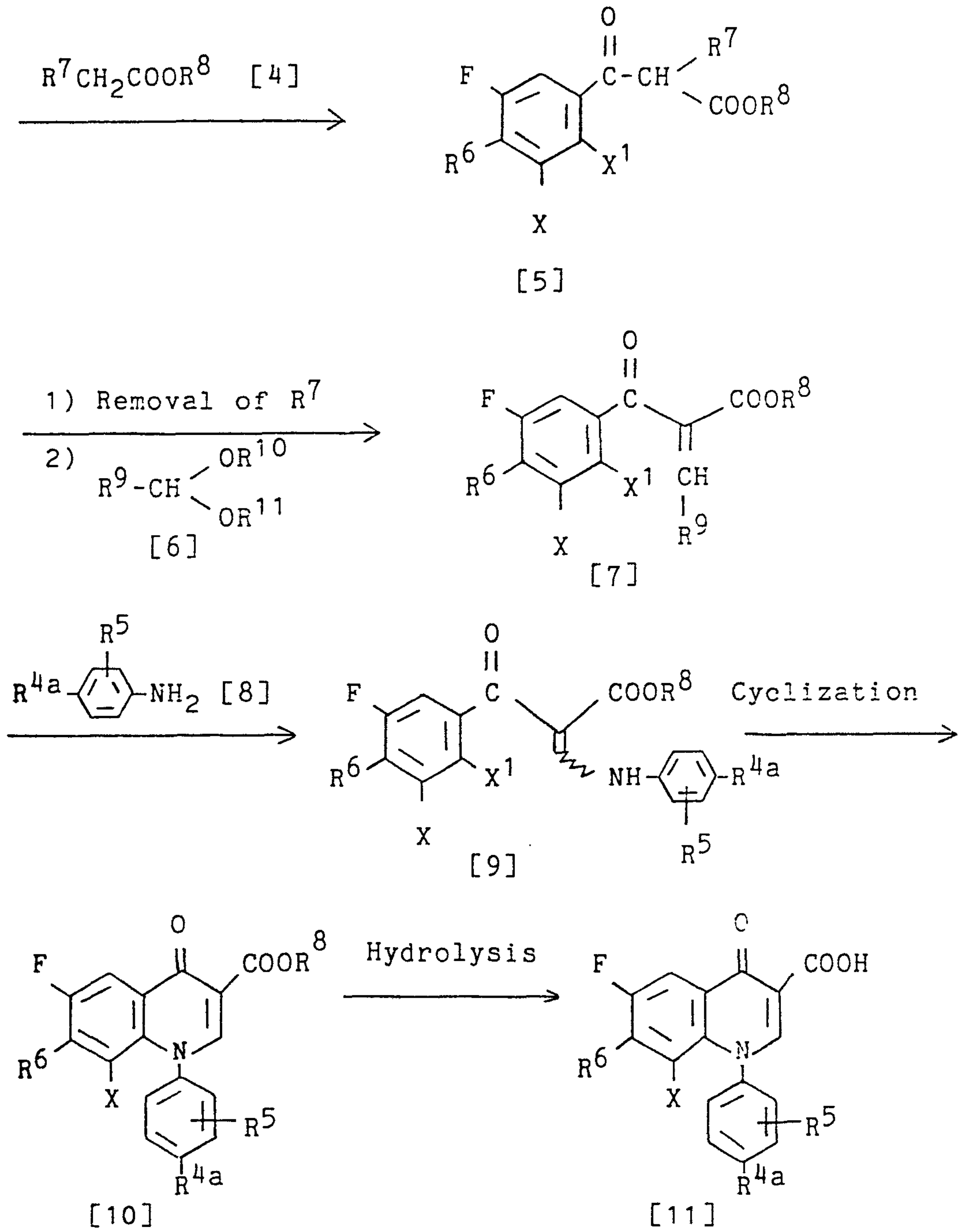


wherein $R^1$, $R^5$ and X are as defined above, $R^{4a}$ is hydroxy, an alkoxy having 1 to 6 carbon atoms or fluorine atom, $R^6$ is halogen atom or a group of the formula: 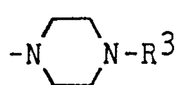 ($R^3$ is as defined above) or a group of the formula: 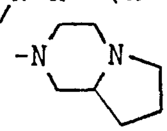 ,

$R^7$ is a group of the formula: $-COR^{12}$ (wherein $R^{12}$ is an alkyl having 1 to 6 carbon atoms) or a group of the formula: $-COOR^{13}$ (wherein $R^{13}$ is an alkyl having 1 to 6 carbon atoms), $R^8$ is an alkyl having 1 to 6 carbon atoms, $R^9$ is a group of the formula: $-N{<}^{R^{14}}_{R^{15}}$ (wherein $R^{14}$ and $R^{15}$ are each an alkyl having 1 to 6 carbon atoms) or an alkoxy having 1 to 6 carbon atoms, and $X^1$ and $X^2$ are each a halogen atom.

The halogenation of the compound [2] is carried out by reacting with a halogenating agent in the presence or absence of a solvent. The solvent includes aromatic hydrocarbons (e.g. benzene, toluene, xylene, etc.), halogenated hydrocarbons (e.g. dichloromethane, chloroform, carbon tetrachloride, etc.), ethers (e.g. dioxane, tetrahydrofuran, diethyl ether, etc.), dimethylformamide (DMF), dimethylsulfoxide (DMSO), and the like. The halogenating agent may be any conventional halogenating agents which can convert hydroxy in carboxy group into a halogen atom, and includes, for example, thionyl chloride, phosphorus oxychloride, phosphorus oxybromide, phosphorus pentachloride, phosphorus pentabromide, and the like. The amounts of the compound [2] and the halogenating agent are not specified, but, in case of using no solvent, the halogenating agent is usually used in a large excess amount, and in case of using a solvent, the halogenating agent is usually used in an amount of at

least 1 mole, preferably 2 to 4 moles, per 1 mole of the compound [2]. The reaction temperature and the reaction period of time are not specified, either, but the reaction is usually carried out at a temperature of from room temperature to 100°C for 30 minutes to 6 hours.

The reaction of the compound [3] and the compound [4] is carried out in a suitable solvent in the presence of a basic compound. The solvent may be any conventional solvents unless they give any undesirable effect on the reaction, and includes, for example, water, ethers (e.g. diethyl ether, dioxane, tetrahydrofuran, monoglyme, diglyme, etc.), alcohols (e.g. methanol, ethanol, isopropanol, etc.), aromatic hydrocarbons (e.g. benzene, toluene, xylene, etc.), aliphatic hydrocarbons (e.g. n-hexane, heptane, cyclohexane, ligroin, etc.), amines (e.g. pyridine, N,N-dimethylaniline, etc.), halogenated hydrocarbons (e.g. chloroform, dichloromethane, carbon tetrachloride, etc.), aprotic polar solvents (e.g. DMF, DMSO, hexamethyl phosphoric triamide (HMPA), etc.), and a mixture of these solvents. The basic compound includes inorganic bases (e.g. metallic sodium, metallic potassium, metallic magnesium, sodium hydride, sodium amide, sodium hydroxide, potassium hydroxide, sodium carbonate, potassium carbonate, sodium hydrogen carbonate, etc.), metal alcoholates (e.g. sodium methylate, sodium ethylate, etc.), and organic bases (e.g. pyridine, piperidine, quinoline, triethylamine, N,N-dimethylaniline, etc.). The reaction is

usually carried out at a temperature of from 0° to 150°C, preferably from room temperature to 120°C, for 0.5 to 15 hours. The compound [4] is usually used in an amount of at least 1 mole, preferably 1 to 2 moles, per 1 mole of the compound [3]. The basic compound is usually used in an amount of at least 1 mole, preferably 1 to 2 moles, per 1 mole of the compound [3].

The compound [5] wherein $R^7$ is the group: $-COR^{12}$ is subjected to the reaction for removal of the group: $-COR^{12}$ in a solvent in the presence of a basic compound. The solvent includes ethers (e.g. diethyl ether, dioxane, tetrahydrofuran, monoglyme, diglyme, etc.), aromatic hydrocarbons (e.g. benzene, toluene, xylene, etc.), aliphatic hydrocarbons (e.g. n-hexane, heptane, cyclohexane, etc.), aprotic polar solvents (e.g. DMF, DMSO, HMPA, etc.), and the like. The basic compound includes ammonia gas, aqueous ammonia, ammonium salts (e.g. ammonium chloride, etc.), primary or secondary amines (e.g. ethylamine, diethylamine, piperidine, etc.), and the like. The reaction is usually carried out at a temperature of from 0° to 150°C, preferably from room temperature to 100°C, for 1 to 20 hours.

The compound [5] wherein $R^7$ is a group: $-COOR^{13}$ is subjected to the reaction for removal of the group: $-COOR^{13}$ in an aqueous solution in the presence of an acid catalyst. The acid catalyst includes mineral acids (e.g. hydrochloric

acid, sulfuric acid, etc.) and organic acids (e.g. p-toluenesulfonic acid, etc.). The reaction is usually carried out at a temperature of from 0° to 150°C, preferably from room temperature to 100°C, for 1 to 20 hours.

The reaction of the $R^7$ group-removed compound and the compound [6] is carried out in a suitable solvent. The solvent may be any solvents which are used in the above reaction for the removal of the $R^7$ group. The reaction is usually carried out at a temperature of from 0° to 150°C, preferably from 0° to 100°C, for 0.5 to 10 hours. The comound [6] is usually used in an equimolar to large excess amount, preferably 1 to 2 moles per 1 mole of the compound [5]. In case of using a compound [6] wherein $R^9$ is a lower alkoxy group, the reaction may also be carried out by using acid anhydrides (e.g. acetic anhydride) as a solvent as well as above-mentioned solvents at a temperature of from 0° to 200°C, preferably 0° to 170°C.

The reaction of the compound [7] and the compound [8] is carried out in a suitable solvent. The solvent may be any conventional solvents unless they give any undesirable effect on the reaction, and includes, for example, alcohols (e.g. methanol, ethanol, propanol), ethers (e.g. diethyl ether, dioxane, tetrahydrofuran, monoglyme, diglyme, etc.), aromatic hydrocarbons (e.g. benzene, toluene, xylene, etc.), aliphatic hydrocarbons (e.g. n-hexane, heptane, cyclohexane, ligroin, etc.), halogenated hydrocarbons (e.g.

chloroform, methylene chloride, carbon tetrachloride, etc.), aprotic polar solvents (e.g. DMF, DMSO, HMPA, etc.), and the like. The reaction is usually carried out at a temperature of from 0° to 150°C, preferably from room temperature to 100°C, for 0.5 to 15 hours. The compound [8] is usually used in an amount of at least 1 mole, preferably 1 to 2 moles, per 1 mole of the compound [7].

The cyclization of the compound [9] is carried out in a suitable solvent in the presence of a basic compound. The solvent may be any conventional solvents unless they give any undesirable effect on the reaction, and includes, for example, ethers (e.g. diethyl ether, dioxane, tetrahydrofuran, monoglyme, diglyme, etc.), aliphatic hydrocarbons (e.g. n-hexane, heptane, ligroin, etc.), halogenated hydrocarbons (e.g. chloroform, methylene chloride, carbon tetrachloride, etc.), aprotic polar solvents (e.g. DMF, DMSO, HMPA, etc.), and the like. The basic compound includes inorganic bases (e.g. metallic sodium, metallic potassium, sodium hydride, sodium amide, sodium hydroxide, potassium hydroxide, etc.), metal alcoholates (e.g. sodium methylate, sodium ethylate, etc.), and organic bases (e.g. 1,8-diazobicyclo[5.4.0]undecene-7 (DBU), N-benzyltrimethylammonium hydroxide, tetrabutylammonium hydroxide, etc.). The reaction is usually carried out at a temperature of from 0° to 150°C, preferably from room temperature to 120°C, for 0.5 to 5 hours. The basic compound is usually used in an

amount of at least 1 mole, preferably 1 to 2 moles, per 1 mole of the compound [9].

The hydrolysis of the compound [10] can be carried out under the conditions of conventional hydrolysis, for instance, in the presence of a basic compound (e.g. sodium hydroxide, potassium hydroxide, barium hydroxide, etc.), a mineral acid (e.g. sulfuric acid, hydrochloric acid, nitric acid, hydrobromic acid, etc.) or an organic acid (e.g. acetic acid, aromatic sulfonic acids, etc.) in a solvent such as water, alcohols (e.g. methanol, ethanol, isopropanol, etc.), ketones (e.g. acetone, methyl ethyl ketone, etc.), ethers (e.g. dioxane, ethylene glycol, etc.), acetic acid, or a mixture thereof. The reaction is usually carried out at a temperature of from room temperature to 200°C, preferably 50° to 150°C, for 0.5 to 6 hours. By the reaction, there is produced the compound [11]. When the compound [10] wherein $R^4a$ is an alkoxy is hydrolyzed with an acid, the ether bond is simultaneously cleaved to give the compound [11] wherein $R^4a$ is hydroxy.

[Reaction Scheme-II]

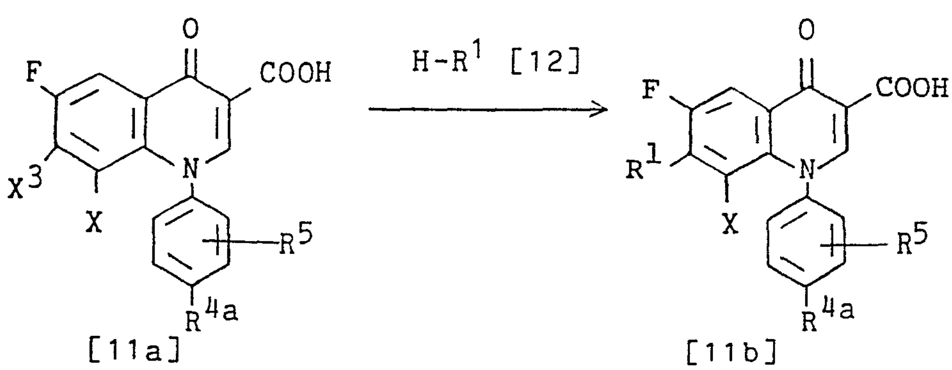


[11a] [11b]

wherein $R^1$, $R^{4a}$, $R^5$ and X are as defined above, and $X^3$ is a halogen atom.

The reaction of the compound [11a] and the compound [12] is carried out in a solvent, wherein both compounds are used in a wide range of ratio, and the compound [12] is usually used in an amount of at least 1 mole, preferably 1 to 5 moles, per 1 mole of the compound [11a]. The solvent includes water, alcohols (e.g. methanol, ethanol, isopropanol, butanol, amyl alcohol, isoamyl alcohol, etc.), aromatic hydrocarbons (e.g. benzene, toluene, xylene, etc.), ethers (e.g. tetrahydrofuran, dioxane, diglyme, etc.), DMF, DMSO, HMPA, N-methylpyrrolidone, or the like. Among these solvents, the preferred one is DMF, DMSO, HMPA, and N-methylpyrrolidone. The reaction may also be carried out in the presence of an acid-removing agent, such as inorganic carbonates (e.g. sodium carbonate, potassium carbonate, sodium hydrogen carbonate, potassium hydrogen carbonate, etc.) or tertiary amines (e.g. pyridine, quinoline, triethylamine, etc.). The reaction is usually carried out under a pressure of from 1 to 20 atm., preferably from 1 to 10 atm., at a temperature of from 100° to 250°C, preferably 100° to 200°C, for 1 to 20 hours.

The compound [11] or [11b] wherein $R^{4a}$ is an alkoxy having 1 to 6 carbon atoms can be converted into the corresponding compound wherein $R^{4a}$ is hydroxy by treating it

with an acid in a solvent or without using any solvent. The solvent includes water, aromatic hydrocarbons (e.g. nitrobenzene, toluene, benzene, etc.), saturated hydrocarbons (e.g. hexane, octane, etc.), lower alcohols (e.g. methanol, ethanol, isopropanol, etc.), ethers (e.g. dioxane, tetrahydrofuran, etc.), ketones (e.g. acetone, etc.), acetic acid, acetonitrile, and a mixture thereof. The acid includes mineral acids (e.g. hydrochloric acid, sulfuric acid, hydrobromic acid, etc.), carboxylic acids (e.g. p-toluenesulfonic acid, pyridine p-toluenesulfonate, acetic acid, propionic acid, etc.), Lewis acids such as alumimum chloride, tin chloride, boron fluoride, zinc chloride, boron tribromide, boron trichloride, and the like. These acids may be used in an amount of at least equimolar to the starting compound, and usually in a large excess amount. The reaction is usually carried out at a temperature of from -30° to 200°C, preferably -30° to 150°C, for 0.5 to 8 hours.

The compound [10] in Reaction Scheme-I wherein $R^{4a}$ is an alkoxy having 1 to 6 carbon atoms can be converted into the correspond compound wherein $R^{4a}$ is hydroxy in the same manner as described above.

The compound [1] wherein $R^4$ is hydroxy can be converted into the corresponding compound wherein $R^4$ is an alkanoyloxy having 1 to 6 carbon atoms by treating it with an alkanoylation agent. The alkanoylation agent includes a

compound of the formula: $(R^{16})_2O$ or $R^{16}X^4$ wherein $R^{16}$ is an alkanoyl having 1 to 6 carbon atoms and $X^4$ is a halogen atom. The reaction can be carried out in the presence or absence of a solvent. The solvent may be any conventional solvents unless they give any undesirable effect on the reaction, and includes, for example, halogenated hydrocarbons (e.g. dichloromethane, dichloroethane, chloroform, carbon tetrachloride), aromatic hydrocarbons (e.g. benzene, toluene, xylene), ethers (e.g. diethyl ether, tetrahydrofuran, dioxane), pyridine, and the like. The alkanoylation agent is used in an amount of at least equimolar to the starting compound, preferably in an excess amount. The reaction is usually carried out at a temperature of from 0° to 150°C, preferably from 0° to 100°C, for 1 to 24 hours. The above reaction may also be carried out in the presence of a basic compound such as a tertiary amine (e.g. pyridine, triethylamine).

[Reaction Scheme-III]

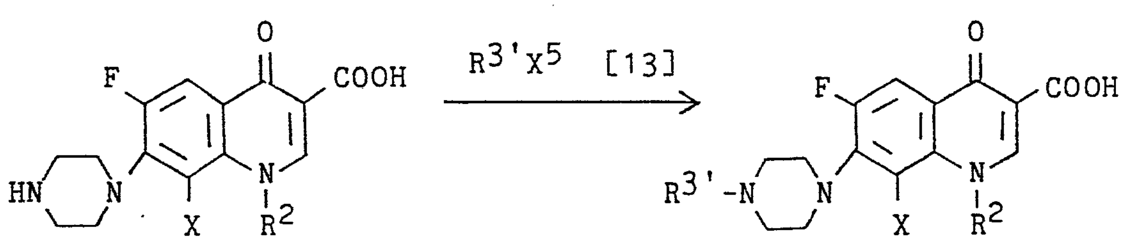


[1a] [1b]

wherein $R^2$ and X are as defined above, $R^{3'}$ is an alkyl having 1 to 2 carbon atoms, and $X^5$ is a halogen atom.

The reaction of the compound [1a] and the compound

[13] is carried out in the presence of a dehydrohalogenating agent in an appropriate solvent. The solvent includes water, alcohols (e.g. methanol, ethanol, isopropanol), ketones (e.g. acetone, methyl ethyl ketone), ethers (e.g. diethyl ether, dioxane), aromatic hydrocarbons (e.g. benzene, toluene, xylene, etc.), and the like. The dehydrohalogenating agent includes inorganic bases (e.g. sodium hydroxide, potassium hydroxide, sodium carbonate, potassium carbonate), alkali metals (e.g. sodium, potassium), and organic bases (e.g. pyridine, piperidine). The compound [13] is used in an amount of at least equimolar to the starting compound [1a] or in excess amount, preferably about 1 to 3 moles to 1 mole of the compound [1a]. The reaction is usually carried out at a temperature of from room temperature to 150°C, preferably at 50° to 120°C, for about 1 to 12 hours.

[Reaction Scheme-IV]

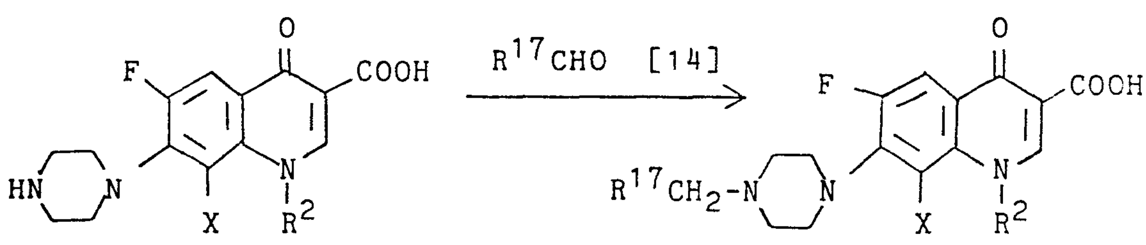


[1a] [1c]

wherein $R^2$ and X are as defined above, $R^{17}$ is hydrogen atom or methyl.

The reaction of the compound [1a] and the compound [14] is carried out in the presence of a reducing agent in

an appropriate solvent or without using any solvent. The solvent includes for example, water, alcohols (e.g. methanol, ethanol, isopropanol), acetic acid, ethers (e.g. dioxane, diethyl ether, diglyme, tetrahydrofuran), aromatic hydrocarbons (e.g. benzene, toluene, xylene, etc.), and the like. The reducing agent includes formic acid, hydroxy reducing agents (e.g. sodium borohydroxide, sodium borocyanohydroxide, aluminum lithium hydroxide), catalytic reducing agents (e.g. palladium black, palladium carbon, platinum oxide, platinum black, Raney nickel) and the like. When formic acid is used as the reducing agent, the reaction is usually carried out at a temperature of from room temperature to 200°C, preferably at 50° to about 150°C, for about 1 to 10 hours. Formic acid is used in an amount of a large excess amount to the compound [1a]. When a hydroxy reducing agent is used, the reaction is usually carried out at a temperature of from -30° to 100°C, preferably at 0° to 70°C, for 30 minutes to 12 hours. The reducing agent is used in an amount of 1 to 20 moles, preferably 1 to 5 moles, per 1 mole of the compound [1a]. Particularly, when aluminum lithium hydroxide is used as the reducing agent, the solvent is preferably ethers (e.g. diethyl ether, dioxane, tetrahydrofuran, diglyme) and aromatic hydrocarbons (e.g. benzene, toluene, xylene). When a catalytic reducing agent is used, the reaction is usually carried out under a pressure of from atmospheric pressure to

20 atm., preferably from atmospheric pressure to 10 atm, at a temperature of from -30° to 100°C, preferably 0° to 60°C, for 1 to 12 hours. The catalyst is usually used in an amount of 0.1 to 40 % by weight, preferably 1 to 20 % by weight, based on the weight of the compound [1a]. The compound [14] is usually used in at least equimolar amount, preferably equimolar to a large excess amount, to the compound [1a].

The compounds [1] can easily be converted into a salt thereof by treating them with a pharmaceutically acceptable acid or base. The acid includes inorganic acids (e.g. hydrochloric acid, sulfuric acid, phosphoric acid, hydrobromic acid, etc.) and organic acids (e.g. succinic acid, maleic acid, fumaric acid, malic acid, tartaric acid, citric acid, lactic acid, benzoic acid, methanesulfonic acid, propionic acid, etc.). The base includes sodium hydroxide, potassium hydroxide, calcium hydroxide, sodium carboante, potasium hydrogen carbonate, and the like.

The compound thus obtained can easily be isolated by conventional methods, such as extraction with solvents, dilution method, recrystallization, column chromatography, preparative thin layer chromatography, and the like.

The compounds of the present invention show particularly excellent antimicrobial activity against Staphylococcus aureus, Staphylococcus pyrogenes, Pseudomonas, anaerobe, various resistant strains of gram

positive or negative bacteria, and hence, are useful as an antimicrobial agent for the treatment of diseases induced by these microorganisms. These compounds show also low toxicity and less side effect and are characteristic in good absobability and in sustained activity. Besides, when the compounds of the present invention are used in the form of a salt, such as lactate, hydrochloride or the like, they show more excellent absorbability.

The compounds of the present invention are usually used in the form of a usual pharmaceutical preparation. The pharmaceutical preparation can be prepared in admixture with conventional pharmaceutically acceptable diluents or carriers, such as fillers, weighting agents, binding agents, wetting agents, disintegrators, surfactants, rublicating agents, and the like. The pharmaceutical preparation includes various preparations suitable for treatment of the diseases, for example, tablets, pills, powders, solutions, suspensions, emulsions, granules, capsules, suppositories, injections (solutions, suspensions, etc.), and the like. In the preparation of tablets, there may be used any conventional carriers, for example, vehicles (e.g. lactose, white sugar, sodium chloride, glucose, urea, starches, calcium carbonate, kaolin, crystalline cellulose, silicate, etc.), binding agents (e.g. water, ethanol, propanol, simple syrup, glucose solution, starch solution, gelatin solution, carboxymethyl cellulose, shellac, methyl cellulose,

potassium phosphate, polyvinylpyrrolidone, etc.), disintegrators (e.g. dry starch, sodium alginate, agar powder, laminaran powder, sodium hydrogen carbonate, calcium carbonate, polyoxyethylene sorbitan fatty acid esters, sodium laurylsulfate, stearic monoglyceride, starches, lactose, etc.), disintegration inhibitors (e.g. white sugar, stearin, cacao butter, hydrogenated oils, etc.), absorption promoters (e.g. quaternary ammonium salts, sodium laurylsulfate, etc.), wetting agents (e.g. glycerin, starches, etc.), adsorbents (e.g. starches, lactose, kaolin, bentonite, colloidal silicates, etc.), rublicants (e.g. purified talc, stearates, boric acid powder, polyethylene glycol, etc.), and the like. The tablets may also be coated with conventional coating agents, for example, may be in the form of a sugar coated tablet, a gelatin-coated tablets, an enteric coating tablet, a film coating tablet, or a double or multiple layers tablet. In the preparation of pills, there may be used conventional carries, such as vehicles (e.g. glucose, lactose, starches, cacao butter, hydrogenated vegetable oils, kaolin, talc, etc.), binding agents (e.g. gum arabic powder, tragacanth powder, gelatin, ethanol, etc.), disintegrators (e.g. laminaran, agar, etc.), and the like. In the preparation of suppositories, there may be used conventional carriers, such as polyethylene glycol, cacao butter, higher alcohols, higher alcohol esters, gelatin, semi-synthetized glycerides, and the like. In the

preparation of injections, the solutions, emulsions or suspensions of the compounds are sterilized and are preferably made isotonic with the body liquid. These solutions, emulsions and suspensions are prepared by admixing the active compound with a conventional diluent, such as water, aqueous lactic acid solution, ethyl alcohol, propylene glycol, ethoxylated isostearyl alcohol, polyoxylated isostearyl alcohol, polyoxyethylene sorbitan fatty acid esters, and the like. The preparations may also be incorporated with sodium chloride, glucose or glycerin in an amount sufficient to make them isotonic with the body liquid. The preparations may also be incorported with conventional solubilizers, buffering agents, anesthetizing agents, and further, with coloring agents, presevatives, perfumes, flavors, sweeting agents, and other medicaments. The preparations in the form of a paste, cream or gel may be prepared by using as a diluent white vaseline, paraffin, glycerin, cellulose derivatives, polyethylene glycol, silicone, bentonite, or the like. Besides, when an active ingredient is precipitated in the injection solution, the precipitate may optionally be dissolved by adding thereto an acid such as methanesulfonic acid, propionic acid, hydrochloric acid, succinic acid, or lactic acid.

The active compounds [1] or salts thereof may be contained in any amount in the preparations, and are usually contained in an amount of 1 to 70 % by weight based on the

whole weight of the preparations.

The pharmaceutical preparations of the present invention can be administered in any methods. Suitable method for administration may be selected in accordance with the preparation form, age and sex of the patients, degree of severity of the diseases, and the like. For instance, tablets, pills, solutions, suspensions, emulsions, granules and capsules are administered in oral route. In case of injection, it is administered intravenously alone or together with an auxiliary liquid (e.g. glucose, amino acid solution). The injections may also be administered in intramuscular, intracutaneous, subcutaneous, or intraperitoneal route. Suppositories are administered in intrarectal route.

The dosage of the pharmaceutical preparations of the present invention may vary according to administration methods, age and sex of the patients, severity of the diseases, and the like, but is usually in the range of about 0.2 to 100 mg of the active compound [1] or a salt thereof per 1 kg of body weight of the patient per day. The preparation is usually administered by dividing into 2 to 4 times per day.

The present invention is illustrated by the following Reference Examples, Examples, Preparations, and Experiments.

Reference Example 1

Magnesium ribbon (0.11 g) is suspended in absolute ethanol (0.5 ml), and thereto is added carbon tetrachloride (0.05 ml). After 10 minutes, to the mixture is added dropwise a mixture of diethyl malonate (1.5 ml), absolute ethanol (0.9 ml) and anhydrous ether (3.8 ml) at a room temperature. After the addition, the mixture is refluxed for 3 hours. Thereafter, a solution of 2,3,4,5-tetrafluorobenzoyl chloride (2.0 g) in anhydrous ether (0.9 ml) is added dropwise under ice-cooling. After the addition, the mixture is stirred at room temperature for 3 hours, and then allowed to stand overnight. A mixture of ice-water (4 ml) and conc. sulfuric acid (0.24 ml) is added dropwise to the reaction mixture under ice-cooling, and the mixture is stirred at room temperature for 30 minutes. The ether layer is separated, and the remaining reaction mixture is extracted with ether. The ether layers are combined, and are washed with saturated aqueous sodium chloride solution and dried over magnesium sulfate. The solvent is distilled off under reduced pressure to give diethyl 2,3,4,5-tetrafluorobenzoylmalonate (3.0 g).

Diethyl 2,3,4,5-tetrafluorobenzoylmalonate (2.9 g) and p-toluenesulfonic acid (15 mg) are added to water (5 ml), and the mixture is refluxed for 3 hours. After cooling, the reaction mixture is extracted with dichloromethane. The dichloromethane layer is washed with saturated

aqueous sodium chloride solution and dried over magnesium sulfate. After distilling off the solvent under reduced pressure, the residue is purified by silica gel column chromatography (solvent, chloroform : n-hexane = 1 : 1) to give ethyl α-(2,3,4,5-tetrafluorobenzoyl)acetate (0.8 g), m.p. 44.5 - 45.5°C.

Reference Example 2

A mixture of ethyl α-(2,3,4,5-tetrafluorobenzoyl)-acetate (0.7 g), ethyl orthoformate (0.67 g) and acetic anhydride (0.74 g) is heated at 150°C for 1.5 hour. After the reaction, the volatile material is distilled off at 120°C under reduced pressure with a water pump to give ethyl 2-(2,3,4,5-tetrafluorobenzoyl)-3-ethoxyacrylate (0.8 g) as a mixture of cis and trans isomers.

NMR ($CDCl_3$) δ: 7.70, 7.75 (s, 1H), 7.28-7.61 (m, 1H) 4.02-4.46 (m, 4H), 1.05-1.52 (m, 6H)

Reference Example 3

A mixture of ethyl 2-(2,3,4,5-tetrafluorobenzoyl)-3-ethoxyacrylate (0.4 g), p-anisidine (0.15 g) and ethanol (4 ml) is stirred at room temperature for 30 minutes. After the reaction, the solvent is distilled off under reduced pressure. To the residue is added anhydrous dioxane (10 ml) and thereto is futher added portionwise 60 % sodium hydride (60 mg). The mixture is stirred at room temperature for 30 minutes. The reaction mixture is poured into saturated aqueous ammonium chloride, and the mixture is extracted with

dichloromethane to give ethyl 6,7,8-trifluoro-1-(4-methoxyphenyl)-1,4-dihydro-4-oxoquinoline-3-carboxylate (0.37 g).

Reference Example 4

A mixture of ethyl 6,7,8-trifluoro-1-(4-methoxyphenyl)-1,4-dihydro-4-oxoquinoline-3-carboxylate (0.37 g), conc. hydrochloric acid (1 ml) and 90 % acetic acid (4 ml) is heated at 120°C for 1 hour. After cooling, the precipitated crystals are separated by filtration, washed with water and with a mixture of ethanol and ether to give 6,7,8-trifluoro-1-(4-methoxyphenyl)-1,4-dihydro-4-oxoquinoline-3-carboxylic acid (0.3 g).

Elementary analysis for $C_{17}H_{10}NO_4F_3$:

Calcd. (%): C,58.46; H,2.89; N,4.01

Found (%): C,58.43; H,2.81, N,4.11.

M.p. 252 - 254°C, white crystals.

Reference Example 5

A mixture of ethyl 6,7,8-trifluoro-1-(4-methoxyphenyl)-1,4-dihydro-4-oxoquinoline-3-carboxylate (0.37 g) and 47 % hydrobromic acid (4 ml) is heated at 120°C for 3 hour. After cooling, the precipitated crystals are separated by filtration, washed with water and with a mixture of ethanol and ether to give 6,7,8-trifluoro-1-(4-hydroxyphenyl)-1,4-dihydro-4-oxoquinoline-3-carboxylic acid (0.26 g).

Elementary analysis for $C_{16}H_8NO_4F_3$:

Calcd. (%): C,57.33; H,2.41; N,4.18

Found (%): C,57.28; H,2.47, N,4.11.

M.p. >300°C, white crystals.

NMR (trifluoroacetic acid) δ: 9.33 (s, 1H), 8.46 (dt, 1H, J=10 Hz, 7.5 Hz, 2.5 Hz) 7.56 (d, 2H, J=9 Hz), 7.27 (d, 2H, J=9 Hz).

Reference Examples 6-7

In the same manner as described in Reference Example 4 by using appropriated statring materials, the following compounds are obtained.

6,7,8-Trifluoro-1-(4-fluorophenyl)-1,4-dihydro-4-oxoquinoline-3-carboxylic acid, m.p. 263 - 266°C, colorless crystals.

6,7,8-Trifluoro-1-(2-fluoro-4-hydroxyphenyl)-1,4-dihydro-4-oxoquinoline-3-carboxylic acid, m.p. >300°C, colorless crystals, NMR (trifluoroacetic acid) δ: 9.31 (1H, s), 8.45 (1H, ddd, J=10 Hz, 7.5 Hz, 2.5 Hz), 7.60 (1H, t, J=7.5 Hz), 7.06 (2H, dd, J=7.5 Hz, 2 Hz).

Reference Example 8

Thionyl chloride (70 ml) is added to 2-bromo-4,5-difluorobenzoic acid (39.5 g), and the mixture is allowed to stand at room temperature for one hour and then refluxed for one hour. After completion of the reaction, the excess thionyl chloride is distilled off under reduced pressure. The oily residue is distilled under reduced pressure to give 2-bromo-4,5-difluorobenzoyl chloride (37.8 g) as a pale yellow oily substance, b.p. 121 - 123°C (32 mmHg).

Reference Example 9

To a mixture of ethyl acetacetate (13.0 g), ligroin (16 ml) and water (32 ml) is added 33 % aqueous sodium hydroxide (4.4 ml) with stirring under ice-cooling. To the mixture are gradually added a solution of 2-bromo-4,5-difluorobenzoyl chloride (25.6 g) in ligroin (14 ml) and 33 % aqueous sodium hydroxide (18 ml) with stirring under ice-cooling. After the addition, the mixture is stirred at room temperature for 14 hours. The precipitated crystals are separated by filtration and washed with water. The fitrate is weakly acidified with ammonium chloride and extracted with dichloromethane. The crystals collected above are dissolved in methanol (200 ml) and thereto is added ammonium chloride (20 g). The mixture is stirred at room temperature for one hour and then extracted with dichloromethane. This extract is combined with the dichloromethane extract obtained above, dired over sodium sulfate, and then the solvent is distilled off to give ethyl 2-acetyl-2-(2-bromo-4,5-difluorobenzoyl)acetate (30.1 g) as a yellow oily substance.

NMR ($CDCl_3$) δ: 17.20 (bs, 1H), 7.38 (dd, 1H, J=9 Hz, 7 Hz), 7.10 (dd, 1H, J=9.5 Hz, 8.5 Hz), 3.97 (q, 2H, J=6 Hz), 2.53 (s, 3H), 1.93 (t, 3H, J=6 Hz)

Reference Example 10

Ethyl 2-acetyl-2-(2-bromo-4,5-difluorobenzoyl)-acetate (24.3 g) is dissolved in anhydrous diethyl ether (50 ml), and thereto is slowly passed ammonia gas under ice-cooling for 45 minutes. The mixture is allowed to stand at room temperature for 17 hours. To the reaction mixture are added dichloromethane and water, and the mixture is acidified with 1N hydrochloric acid and the dichloromethane layer is separated. The dichloromethane layer is washed with saturated aqeuous sodium chloride, dried over sodium sulfate, and the solvent is distilled off. To the residue are added anhydrous benzene (65 ml) and DMF-dimethylacetal (11 ml), and the mixture is refluxed for 2 hours. The solvent is distilled off, and the resulting residue is purified by silica gel column chromatography (solvent, dichloromethane : methanol = 80 : 1) to give ethyl 2-(2-bromo-4,5-difluorobenzoyl)-3-dimethylaminoacrylate (9.0 g) as an orange color oily substance.

NMR ($CDCl_3$) δ: 7.83 (s, 1H), 7.33 (dd, 1H, J=9 Hz, 8.5 Hz), 7.19 (dd, 1H, J=11 Hz, 8.5 Hz), 3.94 (q, 2H, J=7 Hz), 3.30 (bs, 3H), 3.02 (bs, 3H), 1.95 (t, 3H, J=7 Hz)

Reference Example 11

Ethyl 2-(2-bromo-4,5-difluorobenzoyl)-3-dimethylaminoacrylate (2.18 g) and p-anisidine (0.9 g) are dissolved in anhydrous benzene (12 ml), and the mixture is stirred at room temperature for 2 hours. After completion of the reaction, the benzene layer is taken out, washed with diluted hydrochloric acid and saturated aqueous sodium chloride in order, and dried over sodium sulfate. The solvent is distilled off, and the resulting residue is

purified by silica gel column chromatography (solvent, dichloromethane) to give ethyl 2-(2-bromo-4,5-difluorobenzoyl)-3-p-anisidylacrylate (2.09 g) in the form of a mixture of trans and cis isomers (3 : 1) as a yellow sold, m.p. 98 - 104°C.

Reference Example 12

The cis-trans mixture of ethyl 2-(2-bromo-4,5-difluorobenzoyl)-3-p-anisidylacrylate (2.04 g) obtained in Reference Example 4 is dissolved in anhydrous dioxane (40 ml), and thereto is added 60 % sodium hydride (0.24 g), and the mixture is stirred at room temperature for one hour and further refluxed for one hour. After allowing to cool, the reaction mixture is poured into saturated aqueous ammonium chloride under ice-cooling. The precipitated crystals are separated by filtration, washed with water, and then purified by silica gel column chromatography (solvent, dichloromethane : methanol = 80 : 1). The product is recrystallized from ethanol to give ethyl 6,7-difluoro-1-(4-methoxyphenyl)-1,4-dihydro-4-oxoquinoline-3-carboxylate (1.40 g) as colorless needles, m.p. 212 - 214°C.

Reference Example 13

Magnesium ribbon (7.3 g) is suspended in absolute ethanol (15 ml), and thereto is added carbon tetrachloride (1.5 ml), and thereto is added dropwise a mixture of diethyl malonate (48 g), absolute ethanol (30 ml) and anhydrous ether (120 ml) over a period of one hour. After the

addition, the mixture is refluxed for 2 hours. After cooling till room temperature, a solution of 2-bromo-4,5-difluorobenzoyl chloride (92 g) in anhydrous diethyl ether (50 ml) is added dropwise to the mixture. After the addition, the mixture is allowed to stand overnight at room temperature. A mixture of ice-water (120 ml) and conc. sulfuric acid (8 ml) is added dropwise to the reaction mixture under ice-cooling. After the addition, the mixture is extracted with ether. The ether layer is washed with saturated aqueous sodium chloride solution and dried over magnesium sulfate. The solvent is distilled off under reduced pressure to give diethyl (2-bromo-4,5-difluorobenzoyl)malonate (123 g).

Reference Example 14

To a solution of diethyl (2-bromo-4,5-difluorobenzoyl)malonate (75.6 g) in water (100 ml) is added p-toluenesulfonic acid (0.3 g), and the mixture is refluxed for 3 hours. After cooling, the reaction mixture is extracted with dichloromethane. The dichloromethane layer is washed with saturated aqueous sodium chloride solution and dried over magnesium sulfate. After distilling off the solvent under reduced pressure, the residue is distilled to give ethyl (2-bromo-4,5-difluorobenzoyl)acetate (52.0 g), b.p. 105 - 115°C (0.15 mmHg).

Reference Example 15

A mixture of ethyl (2-bromo-4,5-difluorobenzoyl)-

acetate (52.0 g), ethyl orthoformate (36.6 g) and acetic anhydride (41.6 g) is heated at 150°C for 2 hours. After the reaction, the reaction mixture is concentrated under reduced pressure. The resulting residue is purified by silica gel column chromatography (solvent, dichloromethane : n-hexane = 2 : 1) to give ethyl 2-(2-bromo-4,5-difluorobenzoyl)-3-ethoxyacrylate (49 g).

Reference Example 16

Ethyl 2-(2-bromo-4,5-difluorobenzoyl)-3-dimethylaminoacrylate (1.09 g) and 2-fluoro-4-methoxyaniline (0.51 g) are dissolved in benzene (10 ml), and the mixture is allowed to stand at room temperature for 2 hours. The reaction mixture is washed with diluted hydrochloric acid and saturated aqueous sodium chloride, and dried over sodium sulfate. After distilling off the solvent, the residue is dissolved in anhydrous dioxane (20 ml) and thereto is added 60 % sodium hydride (0.14 g). The mixture is stirred at room temperature for one hour and further refluxed for one hour. After cooling, the reaction mixture is poured into saturated aqueous ammonium chloride under ice-cooling. The precipitated crystals are separated by filtration, washed with water, purified by silica gel column chromatography (solvent, dichloromethane : methanol = 80 : 1), and then recrystallized from ethanol to give ethyl 6,7-difluoro-1-(2-fluoro-4-methoxyphenyl)-1,4-dihydro-4-oxoquinoline-3-carboxylate (0.59 g) as pale yellow needles, m.p. 170 -

171°C.

Reference Example 17

To ethyl 6,7-difluoro-1-(4-methoxyphenyl)-1,4-dihydro-4-oxoquinoline-3-carboxylate (0.61 g) are added 90 % acetic acid (8.0 ml) and conc. hydrochloric acid (2.0 ml), and the mixture is heated with stirring at 110°C for 2 hours. After the reaction, the solvent is distilled off under reduced pressure. The resulting residue is washed well with water to give 6,7-difluoro-1-(4-methoxyphenyl)-1,4-dihydro-4-oxoquinoline-3-carboxylic acid (0.54 g) as colorless needles, m.p. 256 - 258°C.

Reference Example 18

In the same manner as described in Reference Example 17 using an appropriate starting material, the following compound is prepared.

6,7-Difluoro-1-(4-fluorophenyl)-1,4-dihydro-4-oxoquinoline-3-carboxylic acid, colorless needles, m.p. 263 - 265°C.

Reference Example 19

To ethyl 6,7-difluoro-1-(4-methoxyphenyl)-1,4-dihydro-4-oxoquinoline-3-carboxylate (0.70 g) is added 48 % hydrobromic acid (8 ml), and the mixture is stirred at 100 - 110°C for 3 hour. After cooling, the precipitated crystals are separated by filtration, washed with water and recrystallized from dichloromethane-ethanol to give 6,7-difluoro-1-(4-hydroxyphenyl)-1,4-dihydro-4-oxoquinoline-3-carboxylic acid (0.57 g) as colorless needles, m.p.288 -

290°C.

Reference Example 20

In the same manner as described in Reference Example 17, the following compounds are prepared.

6-Fluoro-1-(4-methoxyphenyl)-7-(4-methyl-1-piperazinyl)-1,4-dihydro-4-oxoquinoline-3-carboxylic acid, colorless needles, m.p. 236 - 238°C (recrystallized from ethanol)

6,7-Difluoro-1-(2,4-difluorophenyl)-1,4-dihydro-4-oxoquinoline-3-carboxylic acid, colorless needles, m.p. 260 - 265°C.

Reference Example 21

To ethyl 6,7-difluoro-1-(2-fluoro-4-methoxyphenyl)-1,4-dihydro-4-oxoquinoline-3-carboxylate (11.5 g) is added 48 % hydrobromic acid (8 ml), and the mixture is stirred at 110 - 120°C for 3 hours. After cooling, water is added to the reaction mixture, and the precipitated crystals are separated by filtration, washed with water and recrystallized from ethanol to give 6,7-difluoro-1-(2-fluoro-4-hydroxyphenyl)-1,4-dihydro-4-oxoquinoline-3-carboxylic acid (0.87 g) as colorless needles, m.p. 290 - 292°C.

Example 1

6,7,8-Triluoro-1-(4-hydroxyphenyl)-1,4-dihydro-4-oxoquinoline-3-carboxylic acid (0.10 g) is suspended in N-methylpyrrolidone (2 ml), and thereto is added N-methylpiperazine (0.15 g), and the mixture is stirred at 90°C for 30 minutes. After completion of the reaction, the solvent is distilled off under reduced pressure, and the residue is washed with ethanol and recrystallized from DMF to give 6,8-difluoro-1-(4-hydroxyphenyl)-7-(4-methyl-1-piperazinyl)-1,4-dihydro-4-oxoquinoline-3-carboxylic acid (0.08 g) as pale yellowish white crylstals, m.p. >300°C.

Elementary analysis for $C_{21}H_{19}NO_3O_4F_2$:

Calcd. (%): C,60.72; H,4.61; N,10.12

Found (%): C,60.64; H,4.53, N,10.18.

NMR (trifluoroacetic acid) δ: 9.18 (s, 1H), 8.30 (dd, 1H, J=12 Hz, 2.5 Hz), 7.51 (d, 2H, J=9 Hz), 7.25 (d, 2H, J=9 Hz), 3.27 (m, 8H), 3.15 (d, 3H, J=5 Hz)

Examples 2-6

In the same manner as described in Example 1, the compounds as shown in Table 1 are prepared.

Table 1

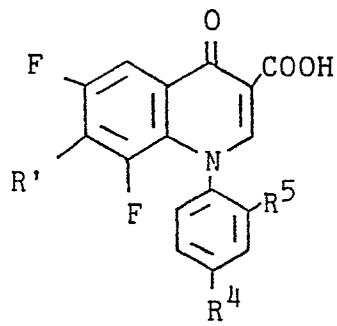

| Ex. No. | R' | $R^5$ | $R^4$ | Crystalline form (Re-(crystalliz. solvent) | m.p. (°C) |
|---|---|---|---|---|---|
| 2 | 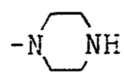 | H | OH | Pale yellowish white crystals (DMF) | >300* |
| 3 | 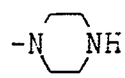 | H | F | Pale yellowish white crystals (DMF) | 281-283 |
| 4 | 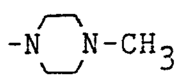 | H | F | Pale yellowish white crystals (Ethanol) | 278-280 |
| 5 | 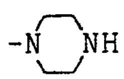 | F | OH | Pale yellowish white crystals (DMF) | 293-297 (dec.) |
| 6 | 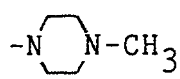 | F | OH | Pale yellowish white crystals (DMF-ethanol) | 298-303 (dec.) |

*) NMR (trifluoroacetic acid) δ: 9.18 (s, 1H), 8.31 (dd, 1H, J=12.5 Hz, 2.5 Hz), 7.60 (brs, 2H), 7.50 (d, 2H, J=12 Hz), 7.26 (d, 2H, J=12 Hz).

Example 7

To a solution of 6,8-Difluoro-1-(4-hydroxyphenyl)-7-(1-piperazinyl)-1,4-dihydro-4-oxoquinoline-3-carboxylic acid (4.18 g) in DMF (29 ml) is added a solution of methyl iodide (7.7 g) in DMF (20 ml). The mixture is heated in a stainless steel autoclave on an oil bath at 110 - 120°C for 5 hours. DMF is distilled off under reduced pressure, and

to the residue is added 10 % aqueous sodium hydroxide to regulate pH 13. The insoluble substance is filtered off, and the remaining mixture is regulated to pH 8 with acetic acid. The precipitated crystals are separated by filtration, washed with water, dried, and then recrystallized from DMF to give 6,8-difluoro-1-(4-hydroxyphenyl)-7-(4-methyl-1-piperazinyl)-1,4-dihydro-4-oxoquinoline-3-carboxylic acid (1.7 g) as pale yellowish white crystals, m.p. >300°C.

Elementary analysis for $C_{21}H_{19}N_3O_4F_2$:

Calcd. (%): C,60.72; H,4.61; N,10.12

Found (%): C,60.66; H,4.70, N,10.18

NMR (trifluoroacetic acid) δ: 9.18 (s, 1H), 8.30 (dd, 1H, J=12 Hz, 2.5 Hz), 7.51 (d, 2H, J=9 Hz), 7.25 (d, 2H, J=9 Hz), 3.27 (m, 8H), 3.15 (d, 3H, J=5 Hz)

In the same manner as described above by using an appropriate stating material, the same compounds as obtained in Examples 4 and 6 are obtained.

Example 8

6,8-Difluoro-1-(4-hydroxyphenyl)-7-(1-piperazinyl)-1,4-dihydro-4-oxoquinoline-3-carboxylic acid (0.80 g) is added slowly to a mixture of 90 % formic acid (4 ml) and 37 % formaline (4 ml) under ice-cooling. After the addition, the mixture is refluxed for 5 hours. The reaction mixture is distilled to dryness under reduced pressure. The residue is suspended in saturated aqueous sodium hydrogen carbonate

(15 ml), and the mixture is stirred at room temperature for 30 minutes. The precipitated crystals are separated by filtration, washed with water, dried, and then recrystallized from DMF to give 6,8-difluoro-1-(4-hydroxyphenyl)-7-(4-methyl-1-piperazinyl)-1,4-dihydro-4-oxoquinoline-3-carboxylic acid (0.52 g) as pale yellowish white crystals, m.p. >300°C.

NMR (trifluroacetic acid) δ: 9.18 (s, 1H), 8.30 (dd, 1H, J=12 Hz, 2.5 Hz), 7.51 (d, 2H, J=9 Hz), 7.25 (d, 2H, J=9 Hz), 3.27 (m, 8H), 3.15 (d, 3H, J=5 Hz).

In the same manner as described above by using an appropriate starting material, there are obtained the same compounds as obtained in Examples 4 and 6.

Example 9

6,8-Difluoro-7-(4-methyl-1-piperazinyl)-1-(4-hydroxyphenyl)-1,4-dihydro-4-oxoquinoline-3-carboxylic acid (0.14 g) is dissolved in dry pyridine (5 ml) and acetic anhydride (5 ml), and the mixture is allowed to stand at room temperature for 16 hours. Excess acetic anhydride and pyridine are distilled off under reduced pressure, and the resulting residue is dissolved in dichloromethane. The dichloromethane solution is washed with diluted hydrochloric acid, saturated aqueous sodium chloride, saturated aqueous sodium hydrogen carbonate and saturated aqeuous sodium chloride in this order, and dried over sodium sulfate. The solvent is distilled off, and the residue is recrystallized

from ethanol-chloroform to give 6,8-difluoro-7-(4-methyl-1-piperazinyl)-1-(4-acetyloxyphenyl)-1,4-dihydro-4-oxo-quinoline-3-carboxylic acid (0.12 g) as pale yellowish white crystals, m.p. 247 - 251°C.

Elementary analysis for $C_{23}H_{21}N_3O_5F_2$:

Calcd. (%): C,60.39; H,4.63; N,9.19

Found (%): C,60.32; H,4.67, N,9.23.

In the same manner as described above by using an appropriate starting material, there is obtained the following compound.

6,8-Difluoro-1-(2-fluoro-4-acetyloxyphenyl)-7-(4-methyl-1-piperazinyl)-1,4-dihydro-4-oxoquinoline-3-carboxylic acid, pale yellowish white crystals, m.p. 224 - 225.5°C (dec.) (recrystallized from ethanol)

Example 10

6,7-Difluoro-1-(4-hydroxyphenyl)-1,4-dihydro-4-oxoquinoline-3-carboxylic acid (0.10 g) is suspended in N-methylpyrrolidone (2 ml), and thereto is added 1,4-diaza-bicyclo[4.3.0]nonane (0.19 g), and the mixture is stirred at 90°C for 30 minutes. After completion of the reaction, the solvent is distilled off under reduced pressure, and the residue is washed with ethanol and recrystallized from DMF to give 6-fluoro-1-(4-hydroxyphenyl)-7-(1,4-diazabicyclo-[4.3.0]nonan-1-yl)-1,4-dihydro-4-oxoquinoline-3-carboxylic acid (0.06 g) as colorless crylstals, m.p. 296-301°C (decomp.)

Elementary analysis for $C_{23}H_{22}N_3O_4F$:

Calcd. (%): C,65.24; H,5.24; N,9.92

Found (%): C,65.20; H,5.27, N,9.94.

Examples 11-13

In the same manner as described in Example 10 using appropriate starting materials, the compounds as shown in Table 2 are prepared.

Table 2

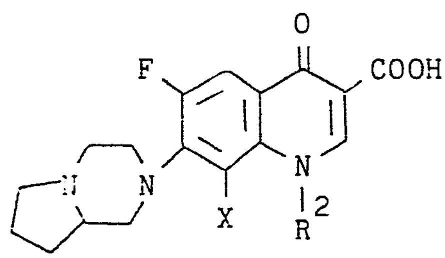

| Ex. No. | X | $R^2$ | M.p. (°C) (Recrys-(talliz. solvent) | Crystalline form |
|---|---|---|---|---|
| 11 | F | 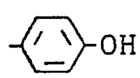 | 298-302 (dec.) (Dimethylformamide) | Pale yellowish white crystals |
| 12 | H | 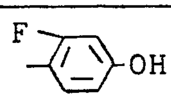 | 270-274 (dec.) (DMF-ethanol) | " |
| 13 | H | 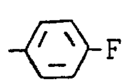 | 262-265 (dec.) (Ethyl acetate) | Pale yellowish white crystals |

Example 14

6-Fluoro-7-(1,4-diazabicyclo[4.3.0]nonan-1-yl)-1-(2-fluoro-4-hydroxyphenyl)-1,4-dihydro-4-oxoquinoline-3-carboxylic acid (0.14 g) is dissolved in dry pyridine (5 ml) and acetic anhydride (5 ml), and the mixture is allowed to stand at room temperature for 16 hours. Excess acetic

anhydride and pyridine are distilled off under reduced pressure, and the residue is dissolved in dichloromethane. The dichloromethane solution is washed with diluted hydrochloric acid, saturated aqueous sodium chloride, saturated aqueous sodium hydrogen carbonate, and saturated aqueous sodium chloride in this order, and dried over sodium sulfate. The solvent is distilled off, and the resulting residue is recrystallized from ethanol to give 6-fluoro-7-(1,4-diazabicyclo[4.3.0]nonan-1-yl)-1-(2-fluoro-4-acetyloxyphenyl)-1,4-dihydro-4-oxoquinoline-3-carboxylic acid (0.10 g) as pale yellowish white crystals, m.p. 206-207°C.

Elementary analysis for $C_{25}H_{23}N_3O_4F_2$:

Calcd. (%): C,62.10; H,4.79; N,8.69

Found (%): C,62.07; H,4.84, N,8.64

Example 15

6-Fluoro-7-(1,4-diazabicyclo[4.3.0]nonan-1-yl)-1-(4-hydroxyphenyl)-1,4-dihydro-4-oxoquinoline-3-carboxylic acid (0.14 g) is dissolved in dry pyridine (5 ml) and acetic anhydride (5 ml), and the mixture is allowed to stand at room temperature for 16 hours. Excess acetic anhydride and pyridine are distilled off under reduced pressure, and the residue is dissolved in dichloromethane. The dichloromethane solution is washed with diluted hydrochloric acid, saturated aqueous sodium chloride, saturated aqueous sodium hydrogen carbonate, and saturated aqueous sodium chloride in this order, and dried over sodium sulfate. The solvent is

distilled off, and the resulting residue is recrystallized from ethanol to give 6-fluoro-7-(1,4-diazabicyclo[4.3.0]-nonan-1-yl)-1-(4-acetyloxyphenyl)-1,4-dihydro-4-oxo-quinoline-3-carboxylic acid (0.12 g) as pale yellowish white crystals, m.p. 224-225.5°C (dec.)

Elementary analysis for $C_{25}H_{24}N_3O_5F$:

Calcd. (%): C,64.50; H,5.20; N,9.03

Found (%): C,64.52; H,5.24, N,8.99

Example 16

6,8-Difluoro-7-(1,4-diazabicyclo[4.3.0]nonan-1-yl)-1-(4-hydroxyphenyl)-1,4-dihydro-4-oxoquinoline-3-carboxylic acid (0.14 g) is dissolved in dry pyridine (5 ml) and acetic anhydride (5 ml), and the mixture is allowed to stand at room temperature for 16 hours. Excess acetic anhydride and pyridine are distilled off under reduced pressure, and the residue is dissolved in dichloromethane. The dichloromethane solution is washed with diluted hydrochloric acid, saturated aqueous sodium chloride, saturated aqueous sodium hydrogen carbonate, and saturated aqueous sodium chloride in this order, and dried over sodium sulfate. The solvent is distilled off, and the resulting residue is recrystallized from ethanol to give 6,8-difluoro-7-(1,4-diazabicyclo[4.3.0]nonan-1-yl)-1-(4-acetyloxyphenyl)-1,4-dihydro-4-oxoquinoline-3-carboxylic acid (0.09 g) as pale yellowish white crystals, m.p. 230-232°C.

Elementary analysis for $C_{25}H_{23}N_3O_5F_2$:

Calcd. (%): C,62.10; H,4.79; N,8.69

Found (%): C,62.13; H,4.82, N,8.71

Example 17

6,7-Difluoro-1-(4-hydroxyphenyl)-1,4-dihydro-4-oxoquinoline-3-carboxylic acid (0.22 g) is suspended in DMSO (10 ml), and thereto is added N-methylpiperazine (0.39 ml), and the mixture is stirred at 110°C for 3.5 hours. After completion of the reaction, the solvent is distilled off under reduced pressure, and the residue is washed with ethanol and recrystallized from DMF to give 6-fluoro-1-(4-hydroxyphenyl)-7-(4-methyl-1-piperazinyl)-1,4-dihydro-4-oxo-quinoline-3-carboxylic acid (0.23 g) as pale yellow prisms, m.p. >300°C.

NMR ($CF_3COOH$) δ: 9.22 (s, 1H), 8.34 (d, 1H, J=12 Hz), 7.48 (d, 2H, J=9 Hz), 7.32 (d, 2H, J=9 Hz), 6.90 (d, 1H, J=6 Hz), 4.20-3.26 (m, 8H), 3.16 (d, 3H, J=5 Hz)

Example 18

6,7-Difluoro-1-(2-fluoro-4-hydroxyphenyl)-1,4-dihydro-4-oxoquinoline-3-carboxylic acid (0.34 g) is suspended in DMSO (10 ml), and thereto is added N-methylpiperazine (0.56 ml), and the mixture is stirred at 105 - 110°C for one hour. After completion of the reaction, the solvent is distilled off under reduced pressure, and the residue is washed with ethanol and recrystallized from DMF to give 6-fluoro-1-(2-fluoro-4-hydroxyphenyl)-7-(4-methyl-1-piperazinyl)-1,4-dihydro-4-oxoquinoline-3-carboxylic acid (0.33 g) as pale yellow powders, m.p. >300°C.

NMR (trifluoroacetic acid) δ: 9.20 (s, 1H), 8.34 (d, 1H, J=13 Hz), 7.52 (t, 1H, J=8.5 Hz), 7.27-7.00 (m, 2H), 6.87 (d, 1H, J=6 Hz), 4.20-3.27 (m, 8H), 3.17 (d, 3H, J=4 Hz)

Example 19

6-Fluoro-7-(4-methyl-1-piperazinyl)-1-(4-hydroxyphenyl)-1,4-dihydro-4-oxoquinoline-3-carboxylic acid (0.13 g) is dissolved in dry pyridine (5 ml) and anhydrous acetic acid (5 ml), and the mixture is allowed to stand at room temperature for 16 hours. Excess amounts of anhydrous acetic acid and pyridine are distilled off under reduced pressure, and the residue is dissolved in dichloromethane. The dichloromethane solution is washed with diluted hydrochloric acid, saturated aqueous sodium chloride solution, saturated aqueous sodium hydrogen carbonate solution and saturated aqueous sodium chloride solution in order, and then dried over sodium sulfate. After distilling off the solvent, the resulting residue is recrystallized from ethanol to give 6-fluoro-7-(4-methyl-1-piperazinyl)-1-(4-acetyloxyphenyl)-1,4-dihydro-4-oxoquinoline-3-carboxylic acid (0.11 g) as pale yellow plates, m.p. 237 - 239°C.

Examples 20 to 23

In the same manner as described in Example 19, the following compounds are prepared.

(20) 6-Fluoro-7-(4-methyl-1-piperazinyl)-1-(4-acetyloxy-2-fluorophenyl)-1,4-dihydro-4-oxoquinoline-3-carboxylic acid, pale yellow needles, m.p. 204 - 206°C

(recrystallized from ethanol)

(21) 6-Fluoro-7-(4-methyl-1-piperazinyl)-1-(4-isobutyryloxypheny)-1,4-dihydro-4-oxoquinoline-3-carboxylic acid, pale yellow plates, m.p. 163 - 165°C (recrystallized from ethanol)

(22) 6-Fluoro-7-(4-methyl-1-piperazinyl)-1-(4-tert.-butylcarbonyloxyphenyl)-1,4-dihydro-4-oxoquinoline-3-carboxylic acid, colorless powdery crystals, m.p. 233 - 235°C (recrystallized from ethanol)

(23) 6-Fluoro-7-(4-ethyl-1-piperazinyl)-1-(4-acetyloxyphenyl)-1,4-dihydro-4-oxoquinoline-3-carboxylic acid, pale yellowish white crystals, m.p. 239 - 242°C (recrystallized from ethanol)

<u>Example 24</u>

In the same manner as described in Example 18, the following compound is prepared.

6-Fluoro-1-(2,4-difluorophenyl)-7-(4-methyl-1-piperazinyl)-1,4-dihydro-4-oxoquinoline-3-carboxylic acid, pale yellow crystals, m.p. 234 - 238°C

<u>Example 25</u>

To 6-fluoro-1-(4-methoxyphenyl)-7-(4-methyl-1-piperazinyl)-1,4-dihydro-4-oxoquinoline-3-carboxylic acid (0.77 g) is added 48 % hydrobromic acid (8 ml), and the mixture is stirred at 100 - 110°C for 3 hours. After allowing to cool, the precipitated crystals are separated by filtration, washed with water, and then suspened in

saturated aqueous sodium hydrogen carbonate solution (15 ml), and the mixture is stirred at room temperature for 30 minutes. The precipitated crystals are separated by filtration, washed with water, and recrystallized from DMF to give 6-fluoro-1-(4-hydroxyphenyl)-7-(4-methyl-1-piperazinyl)-1,4-dihydro-4-oxoquinoline-3-carboxylic acid (0.5 g), as pale yellow prisms, m.p. >300°C.

NMR ($CF_3COOH$) δ: 9.22 (s, 1H), 8.34 (d, 1H, J=12 Hz), 7.48 (d, 2H, J=9 Hz), 7.32 (d, 2H, J=9 Hz), 6.90 (d, 1H, J=6 Hz), 4.20-3.26 (m, 8H), 3.16 (d, 3H, J=5 Hz)

In the same manner as above, the compounds as prepared in Examples 1, 2, 5, 6, 10, 11, and 12 are prepared.

Example 26

6-Fluoro-1-(4-hydroxyphenyl)-7-(1-piperazinyl)-1,4-dihydro-4-oxoquinline-3-carboxylic acid (0.79 g) is slowly added to a mixture of 90 % formic acid (4 ml) and 37 % formalin (4 ml) under ice-cooling. After the addition, the mixture is refluxed for 5 hours. The reaction mixture is evaporated to driness under reduced pressure, and the residue is suspended in saturated aqueous sodium hydrogen carbonate solution (15 ml), and the mixture is stirred at room temperature for 30 minutes. The precipitated crystals are separated by filtration, washed with water, dried, and recrystallized from ethanol to give 6-fluoro-1-(4-hydroxyphenyl)-7-(4-methyl-1-piperazinyl)-1,4-dihydro-4-oxoquinoline-3-carboxylic acid (0.5 g), as pale yellow prisms,

m.p. >300°C.

NMR ($CF_3COOH$) δ: 9.22 (s, 1H), 8.34 (d, 1H, J=12 Hz), 7.48 (d, 2H, J=9 Hz), 7.32 (d, 2H, J=9 Hz), 6.90 (d, 1H, J=6 Hz), 4.20-3.26 (m, 8H), 3.16 (d, 3H, J=5 Hz)

In the same manner as above, the compounds as prepared in Examples 18, 19 and 24 are prepared.

Example 27

A cis-trans mixture of ethyl 2-[4-(1,4-diazabicyclo[4.3.0]nonan-1-yl)-2-bromo-5-fluorobenzoyl]-3-(p-hydroxyanilinyl)acrylate (2.51 g) is dissolved in anhydrous dioxane (40 ml) and thereto is added 60 % sodium hydride (0.24 g). The mixture is stirred at room temperature for one hour and further refluxed for one hour. After allowing to cool, the reaction mixture is poured into saturated aqueous ammonium chloride solution under ice-cooling. The mixture is extracted with dichloromethane, and the extract is washed with water, dried, and then distilled to remove the solvent. The residue is purified by silica gel column chromatography. To the thus obtained ethyl 6-fluoro-7-(1,4-diazabicyclo[4.3.0]nonan-1-yl)-1-(4-hydroxyphenyl)-1,4-dihydro-4-oxoquinoline-3-carboxylate (0.77 g) are added 90 % acetic acid (8.0 ml) and conc. hydrochloric acid (2.0 ml), and the mixture is stirred at 110°C for 2 hours. After the reaction, the reaction mixture is distilled under reduced pressure to remove the solvent. The residue is washed well with water and recrystallized from DMF to give 6-fluoro-1-(4-hydroxyphenyl)-7-(1,4-diazabicyclo[4.3.0]nonan-1-yl)-1,4-dihydro-4-oxoquinoline-3-carboxylic acid (0.68 g), as colorless crystals, m.p. 296 - 301°C (dec.)

In the same manner as described above, the compounds as prepared in Examples 11 to 13 are prepared.

Example 28

A cis-trans mixture of ethyl 2-[4-(4-methyl-1-piperazinyl)-2,3,5-trifluorobenzoyl]-3-(p-hydroxyanilinyl)-acrylate (2.18 g) is dissolved in anhydrous dioxane (40 ml) and thereto is added 60 % sodium hydride (0.24 g). The mixture is stirred at room temperature for one hour and further refluxed for one hour. After allowing to cool, the reaction mixture is poured into saturated aqueous ammonium chloride solution under ice-cooling. The mixture is extracted with dichloromethane, and the extract is washed with water, dried, and then distilled to remove the solvent. The residue is purified by silica gel column chromatography. To the thus obtained ethyl 6,8-difluoro-7-(4-methyl-1-piperazinyl)-1-(4-hydroxyphenyl)-1,4-dihydro-4-oxoquinoline-3-carboxylate (0.73 g) are added 90 % acetic acid (8.0 ml) and conc. hydrochloric acid (2.0 ml), and the mixture is stirred at 110°C for 2 hours. After the reaction, the reaction mixture is distilled under reduced pressure to remove the solvent. The residue is washed well with water and recrystallized from DMF to give 6,8-difluoro-1-(4-hydroxyphenyl)-7-(4-methyl-1-piperazinyl)-1,4-dihydro-4-oxoquinoline-3-carboxylic acid (0.65 g), as pale yellowish

white crystals, m.p. >300°C.

NMR (trifluoroacetic acid) δ: 9.18 (s, 1H), 8.30 (dd, 1H, J=12 Hz, 2.5 Hz), 7.51 (d, 2H, J=9 Hz), 7.25 (d, 2H, J=9 Hz), 3.27 (m, 8H), 3.15 (d, 3H, J=5 Hz)

In the same manner as described above, the compounds as prepared in Examples 2 to 6 are prepared.

Example 29

A mixture of 6,8-difluoro-1-(4-hydroxyphenyl)-7-(4-methyl-1-piperazinyl)-1,4-dihydro-4-oxoquinoline-3-carboxylic acid (0.001 mole), lactic acid (0.002 mole) and water (10 ml) is refluxed for 5 minutes. After filtering in hot state, the precipitated crystals are separated by filtration and recrystallized from water to give 6,8-difluoro-1-(4-hydroxyphenyl)-7-(4-methyl-1-piperazinyl)-1,4-dihydro-4-oxoquinoline-3-carboxylic acid lactate (430 mg), as colorless powdery crystals, m.p. >300°C.

NMR (DMSO-$d_6$) δ: 8.37 (s, 1H), 7.86 (dd, 1H, J=12.5 Hz, J=2 Hz), 7.45 (d, 2H, J=9 Hz), 6.88 (d, 2H, J=9 Hz), 4.02 (q, 1H, J=7 Hz), 3.10-3.33 (4H, m), 2.30-2.53 (4H, m), 2.21 (s, 3H), 1.23 (d, 3H, J=7 Hz)

Examples 30 to 34

In the same manner as described in Example 29, the following compounds are prepared.

(30) 6,8-Difluoro-1-(4-acetyloxyphenyl)-7-(4-methyl-1-piperazinyl)-1,4-dihydro-4-oxoquinoline-3-carboxylic acid lactate, m.p. 201 - 202°C, colorless plates

(recrystallized from ethanol)

(31) 6-Fluoro-1-(4-hydroxyphenyl)-7-(1,4-diazabicyclo[4.3.0]nonan-1-yl)-1,4-dihydro-4-oxoquinoline-3-carboxylic acid lactate, m.p. >300°C, colorless powdery crystals (recrystallized from water), NMR (DMSO-$d_6$) δ: 8.52 (1H, s), 7.90 (1H, d, J=12.5 Hz), 7.45 (2H, d, J=9 Hz), 6.96 (2H, d, J=9 Hz), 6.45 (1H, q, J=8 Hz), 4.00 (1H, q, J=7 Hz), 1.40-3.68 (13H, m), 1.23 (3H, t, J=7 Hz)

(32) 6-Fluoro-1-(4-acetyloxyphenyl)-7-(1,4-diazabicyclo[4.3.0]nonan-1-yl)-1,4-dihydro-4-oxoquinoline-3-carboxylic acid lactate, m.p. 195 - 197°C, colorless plates (recrystallized from ethanol)

(33) 6-Fluoro-1-(4-acetyloxyphenyl)-7-(4-methyl-1-piperazinyl)-1,4-dihydro-4-oxoquinoline-3-carboxylic acid lactate, m.p. 214 - 216°C, colorless plates (recrystallized from ethanol)

(34) 6-Fluoro-1-(2-fluoro-4-acetyloxyphenyl)-7-(4-methyl-1-piperazinyl)-1,4-dihydro-4-oxoquinoline-3-carboxylic acid lactate, m.p. 180 - 181°C, colorless plates (recrystallized from ethanol)

Preparation 1

An injection preparation is prepared from the following components.

| Components | Amount |
|---|---|
| 6,8-Difluoro-1-(4-hydroxyphenyl)-7-(1-piperazinyl)-1,4-dihydro-4-oxoquinoline-3-carboxylic acid | 200 mg |
| Glucose | 250 mg |
| Distilled water for injection | q.s. |
| Totally | 5 ml |

6,8-Difluoro-1-(4-hydroxyphenyl)-7-(1-piperazinyl)-1,4-dihydro-4-oxoquinoline-3-carboxylic acid and glucose are dissolved in distilled water for injection, and the solution is added to a 5 ml ampoule, which is purged with nitrogen gas and then subjected to sterilization at 121°C for 15 minutes to give an injection preparation.

Preparation 2

Film coated tablets are prepared from the following components.

| Components | Amount |
|---|---|
| 6,8-Difluoro-1-(4-hydroxyphenyl)-7-(4-methyl-1-piperazinyl)-1,4-dihydro-4-oxoquinoline-3-carboxylic acid | 100 g |
| Avicel (tradename of microcrystalline cellulose, manufactured by Asahi Chemical, Japan) | 40 g |
| Corn starch | 30 g |
| Magnesium stearate | 2 g |
| TC-5 (tradename of hydroxypropyl methylcellulose, manufactured by Shinetsu Kagaku Kogyo, Japan) | 10 g |

| | |
|---|---|
| Polyethylene glycol 6000 | 3 g |
| Castor oil | 40 g |
| Ethanol | 40 g |

6,8-Difluoro-1-(4-hydroxyphenyl)-7-(4-methyl-1-piperazinyl)-1,4-dihydro-4-oxoquinoline-3-carboxylic acid, Avicel, corn starch and magnesium stearate are mixed and kneaded and the mixture is tabletted using a conventional pounder (R 10 mm) for sugar coating (manufactured by Kikusui Seisakusho Co., Ltd., Japan). The tablets thus obtained are coated with a film coating agent consisting of TC-5, polyethylene glycol 6000, castor oil and ethanol to give film coated tablets.

Preparation 3

An ointment is prepared from the following components.

| Components | Amount |
|---|---|
| 6,8-Difluoro-1-(4-hydroxyphenyl)-7-(4-methyl-1-piperazinyl)-1,4-dihydro-4-oxoquinoline-3-carboxylic acid | 2 g |
| Purified lanolin | 5 g |
| Bleached beeswax | 5 g |
| White vaseline | 88 g |
| Totally | 100 g |

Bleached beeswax is made liquid by heating, and thereto are added 6,8-difluoro-1-(4-hydroxyphenyl)-7-(4-methyl-1-piperazinyl)-1,4-dihydro-4-oxoquinoline-3-carboxylic acid, purified lanolin and while vaseline, and

the mixture is heated until it becomes liquid. The mixture is stirred until it is solidified to give an ointment.

Preparation 4

An injection preparation is prepared from the following components.

| Components | Amount |
|---|---|
| 6,8-Difluoro-1-(4-hydroxyphenyl)-7-(4-methyl-1-piperazinyl)-1,4-dihydro-4-oxoquinoline-3-carboxylic acid | 200 mg |
| Glucose | 250 mg |
| Distilled water for injection | q.s. |
| Totally | 5 ml |

6,8-Difluoro-1-(4-hydroxyphenyl)-7-(4-methyl-1-piperazinyl)-1,4-dihydro-4-oxoquinoline-3-carboxylic acid and glucose are dissolved in distilled water for injection, and the solution is added to a 5 ml ampoule, which is purged with nitrogen gas and then subjected to sterilization at 121°C for 15 minutes to give an injection preparation.

Preparation 5

Film coated tablets are prepared from the following components.

| Components | Amount |
|---|---|
| 6,8-Difluoro-1-(4-hydroxy-2-fluorophenyl)-7-(4-methyl-1-piperazinyl)-1,4-dihydro-4-oxoquinoline-3-carboxylic acid | 100 g |
| Avicel (tradename of microcrystalline cellulose, manufactured by Asahi Chemical, Japan) | 40 g |
| Corn starch | 30 g |

| | |
|---|---|
| Magnesium stearate | 2 g |
| TC-5 (tradename of hydroxypropyl methylcellulose, manufactured by Shinetsu Kagaku Kogyo, Japan) | 10 g |
| Polyethylene glycol 6000 | 3 g |
| Castor oil | 40 g |
| Ethanol | 40 g |

6,8-Difluoro-1-(4-hydroxyphenyl)-7-(4-methyl-1-piperazinyl)-1,4-dihydro-4-oxoquinoline-3-carboxylic acid, Avicel, corn starch and magnesium stearate are mixed and kneaded and the mixture is tabletted using a conventional pounder (R 10 mm) for sugar coating (manufactured by Kikusui Seisakusho Co., Ltd., Japan). The tablets thus obtained are coated with a film coating agent consisting of TC-5, polyethylene glycol 6000, castor oil and ethanol to give film coated tablets.

Preparation 6

An injection preparation is prepared from the following components.

| Components | Amount |
|---|---|
| 6-Fluoro-1-(4-hydroxyphenyl)-7-(1,4-diazabicyclo[4.3.0]nonan-1-yl)-1,4-dihydro-4-oxoquinoline-3-carboxylic acid | 200 mg |
| Glucose | 250 mg |
| Distilled water for injection | q.s. |
| Totally | 5 ml |

6-Fluoro-1-(4-hydroxyphenyl)-7-(1,4-azabicyclo-[4.3.0]nonan-1-yl)-1,4-dihydro-4-oxoquinoline-3-carboxylic

acid and glucose are dissolved in distilled water for injection, and the solution is added to a 5 ml ampoule, which is purged with nitrogen gas and then subjected to sterilization at 121°C for 15 minutes to give an injection preparation.

Preparation 7

Film coated tablets are prepared from the following components.

| Components | Amount |
|---|---|
| 6,8-Difluoro-1-(4-hydroxyphenyl)-7-(1,4-diaza bicyclo[4.3.0]nonan-1-yl)-1,4-dihydro-4-oxo quinoline-3-carboxylic acid | 100 g |
| Avicel (tradename of microcrystalline cellulose, manufactured by Asahi Chemical, Japan) | 40 g |
| Corn starch | 30 g |
| Magnesium stearate | 2 g |
| TC-5 (tradename of hydroxypropyl methylcellulose, manufactured by Shinetsu Kagaku Kogyo, Japan) | 10 g |
| Polyethylene glycol 6000 | 3 g |
| Castor oil | 40 g |
| Ethanol | 40 g |

6,8-Difluoro-1-(4-hydroxyphenyl)-7-(1,4-diaza-bicyclo[4.3.0]nonan-1-yl)-1,4-dihydro-4-oxoquinoline-3-carboxylic acid, Avicel, corn starch and magnesium stearate are mixed and kneaded and the mixture is tabletted using a conventional pounder (R 10 mm) for sugar coating (manufactured by Kikusui Seisakusho Co., Ltd., Japan). The

tablets thus obtained are coated with a film coating agent consisting of TC-5, polyethylene glycol 6000, castor oil and ethanol to give film coated tablets.

Preparation 8

An ointment is prepared from the following components.

| Components | Amount |
|---|---|
| 6-Fluoro-1-(4-hydroxyphenyl)-7-(1,4-diaza bicyclo[4.3.0]nonan-1-yl)-1,4-dihydro-4-oxoquioline-3-carboxylic acid | 2 g |
| Purified lanolin | 5 g |
| Bleached beeswax | 5 g |
| White vaseline | 88 g |
| Totally | 100 g |

Bleached beeswax is made liquid by heating, and thereto are added 6-fluoro-1-(4-hydroxyphenyl)-7-(1,4-diaza-bicyclo[4.3.0]nonan-1-yl)-1,4-dihydro-4-oxoquinoline-3-carboxylic acid, purified lanolin and while vaseline, and the mixture is heated until it becomes liquid. The mixture is stirred until it is solidified to give an ointment.

Preparation 9

An ointment is prepared from the following components.

| Components | Amount |
|---|---|
| 6-Fluoro-1-(4-acetyloxyphenyl)-7-(4-methyl-1-piperazinyl)-1,4-dihydro-4-oxoquinoline-3-carboxylic acid | 2 g |
| Purified lanolin | 5 g |

| | |
|---|---|
| Bleached beeswax | 5 g |
| White vaseline | 88 g |
| Totally | 100 g |

Bleached beeswax is made liquid by heating, and thereto are added 6-fluoro-1-(4-acetyloxyphenyl)-7-(4-methyl-1-piperazinyl)-1,4-dihydro-4-oxoquinoline-3-carboxylic acid, purified lanolin and white vaseline, and the mixture is heated until it becomes liquid. The mixture is stirred until it is solidified to give an ointment.

Preparation 10

An injection preparation is prepared from the following components.

| Components | Amount |
|---|---|
| 6-Fluoro-1-(4-acetyloxyphenyl)-7-(4-methyl-1-piperazinyl)-1,4-dihydro-4-oxoquinoline-3-carboxylic acid (hydrochloride) | 200 mg |
| Glucose | 250 mg |
| Distilled water for injection | q.s. |
| Totally | 5 ml |

6-Fluoro-1-(4-acetyloxyphenyl)-7-(4-methyl-1-piperazinyl)-1,4-dihydro-4-oxoquinoline-3-carboxylic acid (hydrochloride) and glucose are dissolved in distilled water for injection, and the solution is added to a 5 ml ampoule, which is purged with nitrogen gas and then subjected to sterilization at 121°C for 15 minutes to give an injection preparation.

Preparation 11

Film coated tablets are prepared from the following components.

| Components | Amount |
| --- | --- |
| 6-Fluoro-1-(4-acetyloxy-2-fluorophenyl)-7-(4-methyl-1-piperazinyl)-1,4-dihydro-4-oxo-quinoline-3-carboxylic acid | 100 g |
| Avicel (tradename of microcrystalline cellulose, manufactured by Asahi Chemical, Japan) | 40 g |
| Corn starch | 30 g |
| Magnesium stearate | 2 g |
| TC-5 (tradename of hydroxypropyl methylcellulose, manufactured by Shinetsu Kagaku Kogyo, Japan) | 10 g |
| Polyethylene glycol 6000 | 3 g |
| Castor oil | 40 g |
| Ethanol | 40 g |

6-Fluoro-1-(4-acetyloxy-2-fluorophenyl)-7-(4-methyl-1-piperazinyl)-1,4-dihydro-4-oxoquinoline-3-carboxylic acid, Avicel, corn starch and magnesium stearate are mixed and kneaded and the mixture is tabletted using a conventional pounder (R 10 mm) for sugar coating (manufactured by Kikusui Seisakusho Co., Ltd., Japan). The tablets thus obtained are coated with a film coating agent consisting of TC-5, polyethylene glycol 6000, castor oil and ethanol to give film coated tablets.

Preparation 12

An ointment is prepared from the following components.

| Components | Amount |
|---|---|
| 6-Fluoro-1-(4-acetyloxy-2-fluorophenyl)-7-(4-methyl-1-piperazinyl)-1,4-dihydro-4-oxoquinoline-3-carboxylic acid | 2 g |
| Purified lanolin | 5 g |
| Bleached beeswax | 5 g |
| White vaseline | 88 g |
| Totally | 100 g |

Bleached beeswax is made liquid by heating, and thereto are added 6-fluoro-1-(4-acetyloxy-2-fluorophenyl)-7-(4-methyl-1-piperazinyl)-1,4-dihydro-4-oxoquinoline-3-carboxylic acid, purified lanolin and while vaseline, and the mixture is heated until it becomes liquid. The mixture is stirred until it is solidified to give an ointment.

Preparation 13

An injection preparation is prepared from the following components.

| Components | Amount |
|---|---|
| 6-Fluoro-1-(4-acetyloxyphenyl)-7-(4-methyl-1-piperazinyl)-1,4-dihydro-4-oxoquinoline-3-carboxylic acid (lactate) | 60 mg |
| Distilled water for injection | q.s. |
| Totally | 5 ml |

6-Fluoro-1-(4-acetyloxyphenyl)-7-(4-methyl-1-piperazinyl)-1,4-dihydro-4-oxoquinoline-3-carboxylic acid (lactate) is dissolved in distilled water for injection, and the solution is added to a 5 ml ampoule, which is purged with nitrogen gas and then subjected to sterilization at

121°C for 15 minutes to give an injection preparation.

Preparation 14

An injection preparation is prepared from the following components.

| Components | Amount |
|---|---|
| 6,8-Difluoro-1-(4-hydroxyphenyl)-7-(4-methyl-1-piperazinyl)-1,4-dihydro-4-oxoquinoline-3-carboxylic acid (lactate) | 60 mg |
| Distilled water for injection | q.s. |
| Totally | 5 ml |

In the same manner as described in Preparation 13, an injection preparation is prepared.

Preparation 15

An injection preparation is prepared from the following components.

| Components | Amount |
|---|---|
| 6-Fluoro-1-(4-acetyloxy-2-fluorophenyl)-7-(4-methyl-1-piperazinyl)-1,4-dihydro-4-oxoquinoline-3-carboxylic acid (lactate) | 60 mg |
| Distilled water for injection | q.s. |
| Totally | 5 ml |

In the same manner as described in Preparation 13, an injection preparation is prepared.

Preparation 16

An injection preparation is prepared from the following components.

| Components | Amount |
|---|---|
| 6-Fluoro-1-(4-hydroxyphenyl)-7-(4-methyl-1-piperazinyl)-1,4-dihydro-4-oxoquinoline-3-carboxylic acid (lactate) | 60 mg |
| Distilled water for injection | q.s. |
| Totally | 5 ml |

In the same manner as described in Preparation 13, an injection preparation is prepared.

Experiment 1 (Antimicrobial activity in in vitro)

The antimicrobial activity of the test compounds as mentioned below was tested by measuring minimum inhibitory concentration (MIC) by the serial dilution method on agar plate [cf. Chemotherapy, 22, 1126-1128 (1974)]. The microorganisms were used in a concentration of 1 x $10^8$ cells/ml (O.D. 660 mμ, 0.07-0.16) and 1 x $10^6$ cells/ml (100 folds dilution). The results are shown in Table 3.

[Test compound]:

1. 6,8-Difluoro-1-(4-hydroxyphenyl)-7-(4-methyl-1-piperazinyl)-1,4-dihydro-4-oxoquinoline-3-carboxylic acid

2. 6,8-Difluoro-1-(4-fluorophenyl)-7-(1-piperazinyl)-1,4-dihydro-4-oxoquinoline-3-carboxylic acid

3. 6,8-Difluoro-1-(4-acetyloxyphenyl)-7-(4-methyl-1-piperazinyl)-1,4-dihydro-4-oxoquinoline-3-carboxylic acid

4. 6,8-Difluoro-1-(2-fluoro-4-hydroxyphenyl)-7-(4-methyl-1-piperazinyl)-1,4-dihydro-4-oxoquinoline-3-carboxylic acid

5. 6,8-Difluoro-1-(2-fluoro-4-acetyloxyphenyl)-7-(4-methyl-1-piperazinyl)-1,4-dihydro-4-oxoquinoline-3-carboxylic acid

6. 6-Fluoro-1-(4-hydroxyphenyl)-7-(1,4-diazabicyclo[4.3.0]nonan-1-yl)-1,4-dihydro-4-oxoquinoline-3-carboxylic acid

7. 6,8-Difluoro-1-(4-hydroxyphenyl)-7-(1,4-diazabicyclo[4.3.0]nonan-1-yl]-1,4-dihydro-4-oxoquinoline-3-carboxylic acid

8. 6-Fluoro-1-(2-fluoro-4-hydroxyphenyl)-7-(1,4-diazabicyclo[4.3.0]nonan-1-yl)-1,4-dihydro-4-oxoquinoline-3-carboxylic acid

9. 6-Fluoro-1-(2-fluoro-4-acetyloxyphenyl)-7-(1,4-diazabicyclo[4.3.0]nonan-1-yl)-1,4-dihydro-4-oxoquinoline-3-carboxylic acid

10. 6-Fluoro-1-(4-acetyloxyphenyl)-7-(1,4-diazabicyclo[4.3.0]nonan-1-yl)-1,4-dihydro-4-oxoquinoline-3-carboxylic acid

11. 6-Fluoro-1-(4-acetyloxyphenyl)-7-(4-methyl-1-piperazinyl)-1,4-dihydro-4-oxoquinoline-3-carboxylic acid

12. 6-Fluoro-1-(4-isobutyryloxyphenyl)-7-(4-methyl-1-piperazinyl)-1,4-dihydro-4-oxoquinoline-3-carboxylic acid

13. 6-Fluoro-1-(4-acetyloxy-2-fluorophenyl)-7-(4-methyl-1-piperazinyl)-1,4-dihydro-4-oxoquinoline-3-carboxylic acid

14. 6-Fluoro-1-(4-hydroxyphenyl)-7-(4-methyl-1-piperazinyl)-1,4-dihydro-4-oxoquinoline-3-carboxylic acid

15. 6-Fluoro-1-(4-hydroxy-2-fluorophenyl)-7-(4-methyl-1-piperazinyl)-1,4-dihydro-4-oxoquinoline-3-carboxylic acid

16. 6-Fluoro-1-(2,4-difluorophenyl)-7-(4-methyl-1-piperazinyl)-1,4-dihydro-4-oxoquinoline-3-carboxylic acid

A. 6-Fluoro-1-(4-fluorophenyl)-7-(4-methyl-1-piperazinyl)-1,4-dihydro-4-oxoquinoline-3-carboxylic acid (Reference compound, disclosed in Abstracts of The 1984 ICAAC, page 102)

B. 6-Fluoro-1-(4-fluorophenyl)-7-(1-piperazinyl)-1,4-dihydro-4-oxoquinoline-3-carboxylic acid (Reference compound, disclosed in Abstracts of the 1984 ICAAC, page 102)

Table 3

| Test microorganisms | Test Compd. No. 1 | | Test Compd. No. 2 | | Test Compd. No. 3 | |
|---|---|---|---|---|---|---|
| | $1 \times 10^8$ | $1 \times 10^6$ | $1 \times 10^8$ | $1 \times 10^6$ | $1 \times 10^8$ | $1 \times 10^6$ |
| Staphylococcus aureus FDA 209P | <0.048 | <0.048 | 0.39 | 0.195 | 0.195 | 0.195 |
| " pyrogens IID S-23 | 0.781 | 0.39 | 12.5 | 6.25 | 6.25 | 3.12 |
| Escherichia coli NIHJ JC-2 | <0.048 | <0.048 | <0.048 | <0.048 | <0.048 | <0.048 |
| " coli No. 29 | <0.048 | <0.048 | <0.048 | <0.048 | <0.048 | <0.048 |
| Klebsiella pneumoniae NCTC 9632 | <0.048 | <0.048 | <0.048 | <0.048 | 0.195 | 0.097 |
| Proteus inconstans IFO 12930 | 0.195 | 0.097 | 0.39 | 0.195 | 3.12 | 1.56 |
| " morganii IID Kono | 0.097 | <0.048 | 0.097 | 0.048 | 1.56 | 0.781 |
| Serratia marcescens IFO 12648 | <0.048 | <0.048 | 0.195 | 0.097 | 0.781 | 0.39 |
| Acinetobacter calcoaceticus AC-54 | 0.097 | <0.048 | 0.39 | 0.195 | 1.56 | 0.781 |
| Pseudomonas aeruginosa ATCC 10145 | 0.39 | 0.195 | 1.56 | 0.781 | 1.56 | 1.56 |
| " aeruginosa E-2 | 0.195 | 0.097 | 1.56 | 0.781 | 1.56 | 1.56 |
| Bacillus subtilis ATCC 6633 | <0.048 | <0.048 | 0.195 | 0.097 | 0.195 | 0.097 |
| Streptococcus faecalis IFO 12580 | 0.195 | 0.097 | 6.25 | 3.12 | 1.56 | 0.781 |

- to be continued -

Table 3 (Continued)

| Test microorganisms | Test Compd. No. 4 | | Test Compd. No. 5 | |
|---|---|---|---|---|
| | $1 \times 10^8$ | $1 \times 10^6$ | $1 \times 10^8$ | $1 \times 10^6$ |
| Staphylococcus aureus FDA 209P | 0.195 | 0.097 | 0.097 | 0.097 |
| " pyrogens IID S-23 | 1.56 | 1.56 | 1.56 | 1.56 |
| Escherichia coli NIHJ JC-2 | 0.097 | 0.097 | 0.097 | <0.048 |
| " coli No. 29 | 0.195 | 0.097 | 0.097 | <0.048 |
| Klebsiella pneumoniae NCTC 9632 | 0.195 | 0.195 | 0.097 | 0.097 |
| Proteus inconstans IFO 12930 | 3.12 | 1.56 | 3.12 | 1.56 |
| " morganii IID Kono | 1.56 | 0.781 | 1.56 | 0.781 |
| Serratia marcescens IFO 12648 | 0.781 | 0.781 | 0.781 | 0.781 |
| Acinetobacter calcoaceticus AC-54 | 0.781 | 0.781 | 0.781 | 0.39 |
| Pseudomonas aeruginosa ATCC 10145 | 3.12 | 1.56 | 3.12 | 1.56 |
| " aeruginosa E-2 | 3.12 | 1.56 | 1.56 | 1.56 |
| Bacillus subtilis ATCC 6633 | 0.39 | 0.195 | 0.39 | 0.195 |
| Streptococcus faecalis IFO 12580 | 0.781 | 0.781 | 1.56 | 0.781 |

- to be continued -

Table 3 (Continued)

| Test microorganisms | Test Compd. No. 6 | | Test Compd. No. 7 | | Test Compd. No. 8 | |
|---|---|---|---|---|---|---|
| | $1 \times 10^8$ | $1 \times 10^6$ | $1 \times 10^8$ | $1 \times 10^6$ | $1 \times 10^8$ | $1 \times 10^6$ |
| Staphylococcus aureus FDA 209P | <0.048 | <0.048 | 0.097 | 0.097 | 0.097 | 0.097 |
| " pyrogens IID S-23 | 0.78 | 0.39 | 3.12 | 0.781 | 0.781 | 0.39 |
| Escherichia coli NIHJ JC-2 | 0.097 | <0.048 | 0.097 | 0.097 | 0.097 | 0.097 |
| " coli No. 29 | <0.048 | <0.048 | 0.097 | <0.048 | 0.195 | 0.097 |
| Klebsiella pneumoniae NCTC 9632 | <0.097 | 0.097 | 0.097 | 0.097 | <0.048 | 0.097 |
| Proteus inconstans IFO 12930 | 1.56 | 0.78 | 1.56 | 1.56 | 1.56 | 0.781 |
| " morganii IID Kono | 0.39 | 0.195 | 0.39 | 0.39 | 0.781 | 0.781 |
| Serratia marcescens IFO 12648 | 0.39 | 0.195 | 0.39 | 0.39 | 0.781 | 0.781 |
| Acinetobacter calcoaceticus AC-54 | 0.39 | 0.195 | 0.39 | 0.195 | 0.781 | 0.195 |
| Pseudomonas aeruginosa ATCC 10145 | 1.56 | 0.78 | 3.12 | 1.56 | 1.56 | 1.56 |
| " aeruginosa E-2 | 1.56 | 0.78 | 1.56 | 1.56 | 1.56 | 1.56 |
| Bacillus subtilis ATCC 6633 | <0.048 | <0.049 | 0.097 | 0.097 | 0.195 | 0.097 |
| Streptococcus faecalis IFO 12580 | 0.78 | 0.39 | 1.56 | 0.781 | 0.781 | 0.39 |

- to be continued -

Table 3 (Continued)

| Test microorganisms | Test Compd. No. 9 | | Test Compd. No. 10 | |
|---|---|---|---|---|
| | $1 \times 10^8$ | $1 \times 10^6$ | $1 \times 10^8$ | $1 \times 10^6$ |
| Staphylococcus aureus FDA 209P | <0.048 | <0.048 | 0.097 | <0.048 |
| " pyrogens IID S-23 | 0.39 | 0.39 | 0.781 | 0.781 |
| Escherichia coli NIHJ JC-2 | 0.097 | 0.097 | 0.097 | 0.097 |
| " coli No. 29 | <0.048 | <0.048 | 0.097 | <0.048 |
| Klebsiella pneumoniae NCTC 9632 | 0.097 | 0.097 | 0.097 | 0.097 |
| Proteus inconstans IFO 12930 | 1.56 | 0.781 | 1.56 | 0.781 |
| " morganii IID Kono | 0.781 | 0.39 | 0.39 | 0.39 |
| Serratia marcescens IFO 12648 | 0.781 | 0.781 | 0.781 | 0.39 |
| Acinetobacter calcoaceticus AC-54 | 0.39 | 0.195 | 0.39 | 0.195 |
| Pseudomonas aeruginosa ATCC 10145 | 1.56 | 1.56 | 1.56 | 1.56 |
| " aeruginosa E-2 | 1.56 | 0.781 | 1.56 | 0.781 |
| Bacillus subtilis ATCC 6633 | 0.097 | 0.097 | 0.097 | <0.048 |
| Streptococcus faecalis IFO 12580 | 0.39 | 0.39 | 0.781 | 0.39 |

- to be continued -

Table 3 (Continued)

| Test microorganisms | Test Compd. No. 11 | | Test Compd. No. 12 | | Test Compd. No. 13 | |
|---|---|---|---|---|---|---|
| | $1 \times 10^8$ | $1 \times 10^6$ | $1 \times 10^8$ | $1 \times 10^6$ | $1 \times 10^8$ | $1 \times 10^6$ |
| Staphylococcus aureus FDA 209P | 0.097 | 0.097 | 0.195 | 0.097 | 0.097 | <0.048 |
| " pyrogens IID S-23 | 1.56 | 0.78 | 3.12 | 1.56 | 0.39 | 0.195 |
| Escherichia coli NIHJ JC-2 | 0.097 | 0.097 | 0.195 | 0.195 | 0.097 | <0.048 |
| " coli No. 29 | 0.097 | <0.048 | 0.097 | 0.097 | <0.048 | <0.048 |
| Klebsiella pneumoniae NCTC 9632 | 0.195 | 0.097 | 0.39 | 0.195 | 0.097 | <0.048 |
| Proteus inconstans IFO 12930 | 1.56 | 0.78 | 3.12 | 1.56 | 0.78 | <0.048 |
| " morganii IID Kono | 0.78 | 0.39 | 1.56 | 0.78 | 0.78 | 0.39 |
| Serratia marcescens IFO 12648 | 0.78 | 0.39 | 1.56 | 0.78 | 0.39 | 0.195 |
| Acinetobacter calcoaceticus AC-54 | 0.78 | 0.39 | 0.78 | 0.78 | 0.39 | 0.195 |
| Pseudomonas aeruginosa ATCC 10145 | 0.78 | 0.78 | 1.56 | 1.56 | 1.56 | 0.78 |
| " aeruginosa E-2 | 1.56 | 0.78 | 1.56 | 1.56 | 0.78 | 0.78 |
| Bacillus subtilis ATCC 6633 | 0.097 | <0.048 | 0.195 | 0.097 | 0.097 | 0.097 |
| Streptococcus faecalis IFO 12580 | - | - | - | - | - | - |

- to be continued -

Table 3 (Continued)

| Test microorganisms | Test Compd. No. 14 | | Test Compd. No. 15 | | Test Compd. No. 16 | |
|---|---|---|---|---|---|---|
| | $1 \times 10^8$ | $1 \times 10^6$ | $1 \times 10^8$ | $1 \times 10^6$ | $1 \times 10^8$ | $1 \times 10^6$ |
| Staphylococcus aureus FDA 209P | 0.097 | <0.048 | 0.097 | 0.048 | 0.097 | 0.097 |
| " pyrogens IID S-23 | 1.56 | 0.78 | 0.78 | 3.12 | 1.56 | 0.78 |
| Escherichia coli NIHJ JC-2 | 0.097 | 0.097 | 0.097 | 0.048 | 0.097 | 0.097 |
| " coli No. 29 | 0.097 | <0.048 | 0.048 | 0.048 | <0.048 | <0.048 |
| Klebsiella pneumoniae NCTC 9632 | 0.097 | <0.048 | 0.195 | 0.097 | 0.097 | 0.097 |
| Proteus inconstans IFO 12930 | 0.78 | 0.39 | 1.56 | 0.78 | 1.56 | 0.78 |
| " morganii IID Kono | 0.39 | 0.39 | 0.78 | 0.78 | 0.39 | 0.39 |
| Serratia marcescens IFO 12648 | 0.39 | 0.195 | 0.78 | 0.39 | 0.78 | 0.39 |
| Acinetobacter calcoaceticus AC-54 | 0.39 | 0.195 | 0.78 | 0.39 | 0.195 | 0.097 |
| Pseudomonas aeruginosa ATCC 10145 | 1.56 | 0.78 | 0.78 | 0.78 | 3.12 | 1.56 |
| " aeruginosa E-2 | 0.78 | 0.39 | 0.78 | 0.78 | 1.56 | 1.56 |
| Bacillus subtilis ATCC 6633 | 0.097 | 0.097 | 0.195 | 0.195 | <0.048 | <0.048 |
| Streptococcus faecalis IFO 12580 | - | - | 0.78 | 0.39 | - | - |

- to be continued -

Table 3 (Continued)

| Test microorganisms | Test Compd. A | | Test Compd. B | |
|---|---|---|---|---|
| | $1 \times 10^8$ | $1 \times 10^6$ | $1 \times 10^8$ | $1 \times 10^6$ |
| Staphylococcus aureus FDA 209P | 0.39 | 0.195 | 0.39 | 0.195 |
| " pyrogens IID S-23 | 6.25 | 3.12 | 6.25 | 3.12 |
| Escherichia coli NIHJ JC-2 | 0.195 | 0.195 | 0.097 | <0.048 |
| " coli No. 29 | 0.195 | 0.097 | 0.097 | <0.048 |
| Klebsiella pneumoniae NCTC 9632 | 0.195 | 0.195 | 0.097 | <0.048 |
| Proteus inconstans IFO 12930 | 3.12 | 3.12 | 0.78 | 0.78 |
| " morganii IID Kono | 0.78 | 0.78 | 0.195 | 0.195 |
| Serratia marcescens IFO 12648 | 3.12 | 0.78 | 0.39 | 0.195 |
| Acinetobacter calcoaceticus AC-54 | 0.39 | 0.39 | 0.39 | 0.195 |
| Pseudomonas aeruginosa ATCC 10145 | 3.12 | 3.12 | 1.56 | 0.78 |
| " aeruginosa E-2 | 3.12 | 3.12 | 0.78 | 0.78 |
| Bacillus subtilis ATCC 6633 | 0.195 | 0.097 | 0.195 | 0.097 |
| Streptococcus faecalis IFO 12580 | - | - | - | - |

<u>Experiment 2</u> (Absorption by oral administration in monkey)

The test compounds were dissolved or suspended in the solvent as mentioned in Table 4, i.e. in 0.1 N hydrochloric acid (abbrevitated as 0.1N HCl), 15 % aqueous lactic acid (abbreviated as 15 % Lact. acid) or 0.5 % sodium carboxymethyl cellulose solution (abbreviated as 0.5 % CMC). The solution or suspension was administered into stomach of monkey (two monkeyes) with cathetel. After the administration of test compounds, the blood was collected from the lower limbs vein at a fixed interval with a syringe treated with heparin. The collected blood was centrifuged at 3,000 r.p.m. for 10 minutes to separate the plasma. The concentration of the test compound was measured by a thin layer cup method (a bio-assay), wherein <u>Bacillus subtilis</u> ATCC 6633 was used as a bacteria for determination. Based on a standard curve, the concentration of test compound in plasma was calculated. The results are shown in Table 4.

Table 4

| Test compd. No. | Solvent | Dose (mg/kg) | Run No. | Amount of test compd. in plasma (γ/ml) Time (hour) 0.5 | 1 | 2 | 4 | 7 | 24 |
|---|---|---|---|---|---|---|---|---|---|
| 1 | 0.1N HCl | 10 | 1 | 2.35 | 4.26 | 1.94 | 0.72 | N.D.*1 | N.D.*1 |
| | | | 2 | N.D.*1 | 2.18 | 1.12 | 1.12 | N.D.*1 | N.D.*1 |
| | | | Average | <1.37 | 3.22 | 1.53 | 0.92 | <0.39 | <0.39 |
| 10 | 15% Lact. acid | 90 | 1 | N.D.*2 | 0.632 | 0.752 | 2.340 | N.D.*2 | N.D.*2 |
| | | | 2 | N.D.*2 | 0.926 | 1.060 | 1.294 | 1.620 | N.D.*2 |
| | | | Average | <0.1 | 0.779 | 0.906 | 1.817 | <0.86 | <0.1 |
| 11 | 0.1N HCl | 100 | 1 | 2.32 | 2.04 | 6.82 | 4.88 | 1.40 | N.D.*3 |
| | | | 2 | 0.20 | 0.64 | 2.20 | 22.24 | 4.38 | N.D.*3 |
| | | | Average | 1.26 | 1.34 | 4.51 | 13.56 | 2.89 | <0.2 |
| 13 | 0.1N HCl | 50 | 1 | 0.23 | 0.25 | 1.58 | 0.36 | 0.27 | 0.32 |
| | | | 2 | 0.91 | 2.01 | 1.30 | 0.33 | 0.30 | 0.31 |
| | | | Average | 0.57 | 1.13 | 1.44 | 0.35 | 0.29 | 0.32 |
| 14 | 0.5% CMC | 100 | 1 | 0.36 | 0.49 | 0.65 | 0.96 | 0.95 | 0.90 |
| | | | 2 | N.D.*3 | N.D.*3 | N.D.*3 | N.D.*3 | N.D.*3 | N.D.*3 |
| | | | Average | <0.28 | <0.35 | <0.43 | <0.58 | <0.58 | <0.55 |

[Note]: N.D.*1: <0.39 γ/ml, N.D.*2: <0.1 γ/ml, N.D.*3: <0.2 γ/ml

When the average was counted in case of N.D.*1, N.D.*2 and N.D.*3, it was counted as 0.39 γ/ml, 0.1 γ/ml or 0.2 γ/ml, respectively, and the average value was shown with a head of "<" (less than)

Experiment 3 (Antimicrobial activity in mice)

E. coli No. 29 strain was cultured in a nutrient medium at 37°C, and the culture solution was diluted with the same medium and thereto was added an equivolume of 6 % mucin to give a cell solution.

The cell solution was intraperitoneally administered to mice (one group: 10 mice) in an amount of 0.5 ml (E. coli cells: $10^5$ - $10^6$ cells per each mouse). One hour after the infection, the test compound was orally administered to the mice, and the mice were observed for one week. Based on the number of died and live mice, there was calculated the 50 % effective dose ($ED_{50}$) of test compounds by Probit method. The results are shown in Table 5.

Table 5

| Test compounds | $ED_{50}$ (mg/kg) |
| --- | --- |
| 11 | 5.28 |
| 14 | 11.75 |

Experiment 4

A lactate of Compound 11 was dissolved in water to give a solution of the lactate (concentration of lactate: 12 mg/ml). Separately Compound 11 was suspended in 0.5 % aqueous sodium carboxymethyl cellulose solution (concentration of Compound 11: 12 mg/ml).

Rats (one group: 3 rats) were subjected to laparotomy, and the solution or suspension of the test compounds as prepared above was administered into duodenal tract in a dose of the test compound of 60 mg/kg. After the administration, each three rats were killed at an interval of one hour, and blood was collected from vena cava inferior with heparine-treated syringe. The blood thus collected was centrifuged at 3,000 r.p.m. for 10 minutes, and the plasma was separated. The cencentration of test compounds in blood plasma was measured by Thin Layer Cup method (bio-assay method) (detecting microorganism: Bacilus subtilis ATCC 6633). The results are shown in Table 6 (the data are in average of three rats).

Table 6

| Test compounds | Dose (mg/kg) | Medium | Amount of test compd. in plasma (μg/ml) | | | | |
|---|---|---|---|---|---|---|---|
| | | | Time (hour) 0.25 | 0.5 | 1 | 2 | 4 |
| 11 (free) | 60 | CMC | 4.6 | 3.1 | 1.0 | 0.6 | - |
| 11 (lactate) | 60 | Water | 13.9 | 4.7 | 1.8 | 0.4 | - |

Experiment 5

The test compounds were intravenously administered to mice and the half lethal dose ($LD_{50}$) was measured. The results are shown in Table 7.

Table 7

| Compound Ncs. | $LD_{50}$ (mg/mg) |
| --- | --- |
| 1 | 160 |
| 6 | 225 |
| 13 | 240 |
| 14 | 225 |

Claims

1. Compounds of the formula

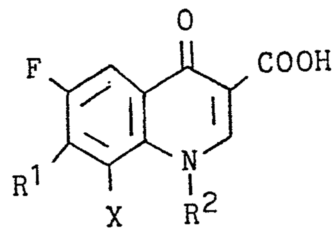

wherein $R^1$ is a group of the formula 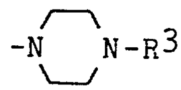

(where $R^3$ is hydrogen atom or an alkyl having 1 to 2 carbon atoms), or a group of the formula 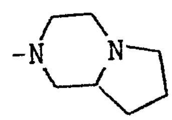 ,

$R^2$ is a group of the formula 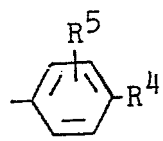

$R^4$ is hydroxy, fluorine atom or an alkanoyloxy having 1 to 6 carbon atoms, $R^5$ is hydrogen atom or fluorine atom, and X is hydrogen atom or fluorine atom, provided that when $R^1$ is 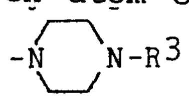

and X is hydrogen atom, $R^2$ is not a group of the formula 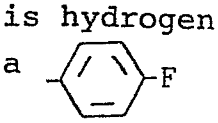

or 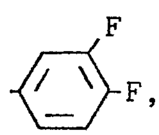

and pharmaceutically acceptable salts thereof.

2. Compounds according to claim 1 which are compounds of the formula

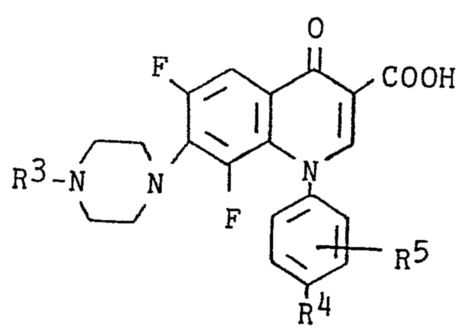

wherein $R^3$ is hydrogen atom or an alkyl having 1 to 2 carbon atoms, $R^4$ is hydroxy, fluorine atom or an alkanoyloxy having 1 to 6 carbon atoms, and $R^5$ is hydrogen atom or fluorine atom, or a pharmaceutically acceptable salt thereof.

3. Compounds according to claim 1 which are compounds of the formula

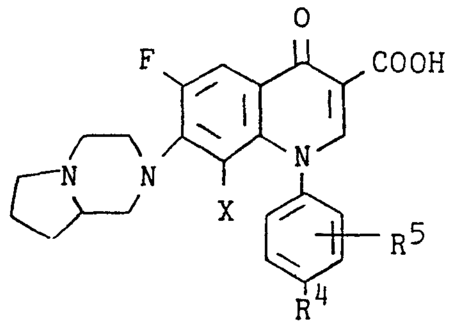

wherein $R^4$ is hydroxy, fluorine atom or an alkanoyloxy having 1 to 6 carbon atoms, $R^5$ is hydrogen atom or fluorine atom, and X is hydrogen atom or fluorine atom, or a pharmaceutically accetable salt thereof.

4. Compounds according to claim 1 which are compounds of the formula

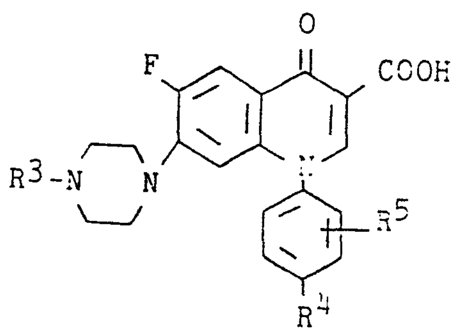

wherein $R^3$ is an alkyl having 1 to 2 carbon atoms, $R^4$ is an alkanoyloxy having 1 to 6 carbon atoms, and $R^5$ is hydrogen atom or fluorine atom, or a pharmaceutically acceptable salt thereof.

5. Compounds according to claim 1 which are compounds of the formula

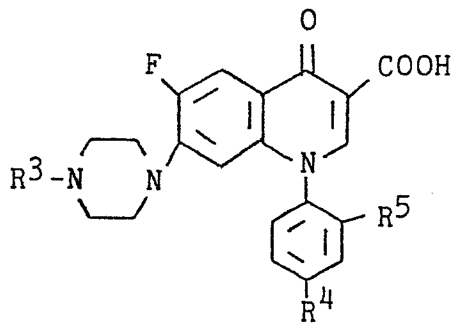

wherein $R^3$ is an alkyl having 1 to 2 carbon atoms, $R^4$ is hydroxy or fluorine atom, and $R^5$ is hydrogen atom or fluorine atom, or a pharmaceutically acceptable salt thereof.

6. Compounds according to anyone of claims 1 to 5, wherein $R^3$ is methyl group, and $R^4$ is hydroxy or an alkanoyloxy having 1 to 6 carbon atoms.

7. Compounds according to anyone of claims 1 to 6, wherein $R^5$ is hydrogen atom.

8. Compounds according to anyone of claims 1 to 7, wherein $R^4$ is hydroxy or acetyloxy group.

9. Compounds according to anyone of claims 1 to 8, wherein $R^5$ is fluorine atom.

10. Compounds according to anyone of claims 1 to 9, wherein $R^5$ is substituted at 2-position on the phenyl ring.

11. Compounds according to anyone of claims 1 to 10, wherein $R^4$ is hydroxy or an alkanoyloxy having 1 to 6 carbon atoms.

12. Compounds according to anyone of claims 1 to 11, wherein $R^3$ is methyl group.

13. Compounds according to anyone of claims 1 to 12, wherein $R^4$ is an alkanoyloxy having 2 to 4 carbon atoms.

14. Compounds according to anyone of claims 1 to 5 selected from 6-fluoro-1-(4-hydroxyphenyl)-7-(1,4-diazabicyclo(4.3.0)nonan-1-yl)-1,4-dihydro-4-oxo-quinoline-3-carboxylic acid or a pharmaceutically acceptable salt thereof; 6-fluoro-1-(4-acetyloxyphenyl)-7-(1,4-diazabicyclo-(4.3.0)nonan-1-yl)-1,4-dihydro-4-oxoquinoline-3-carboxylic acid or a pharmaceutically acceptable salt thereof; 6,8-difluoro-1-(4-hydroxyphenyl)-7-(4-methyl-1-piperazinyl)-1,4-dihydro-4-oxoquinoline-3-carboxylic acid or a pharmaceutically acceptable salt thereof; 6,8-difluoro-1-(4-acetyloxyphenyl)-7-(4-methyl-1-piperazinyl)-1,4-dihydro-4-oxoquinoline-3-carboxylic acid or a pharmaceutically acceptable salt thereof; 6-fluoro-1-(4-acetyloxyphenyl)-7-(4-methyl-1-piperazinyl)-1,4-dihydro-4-oxoquinoline-3-carboxylic acid or a pharmaceutically acceptable salt thereof; and 6-fluoro-1-(4-acetyloxy- 2-fluorophenyl)-7-(4-methyl-1-piperazinyl)-1,4-dihydro-4-oxoquinoline-3-carboxylic acid or a pharmaceutically acceptable salt thereof.

15. An antimicrobial composition which comprises at last one compound of claims 1 to 14 or a pharmaceutically acceptable salt thereof in admixture with a pharmaceutically acceptable diluent or carrier.

16. A process for preparing a compound according to claims 1 to 14 which comprises
(a) subjecting a compound of the formula

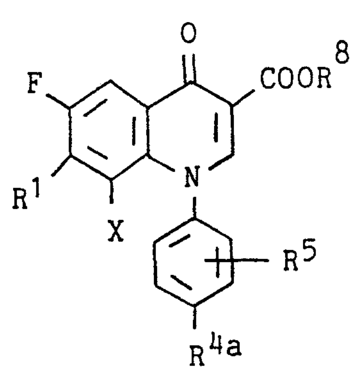

wherein $R^1$, $R^5$ and X are as defined above, $R^{4a}$ is hydroxy, an alkoxy having 1 to 6 carbon atoms or fluorine atom, and $R^8$ is an alkyl having 1 to 6 carbon atoms, to hydrolysis, optionally followed by subjecting to conversion of alkoxy in $R^{4a}$ into hydroxy and further optionally to alkanoylation of hydroxy, to obtain a compound of the formula:

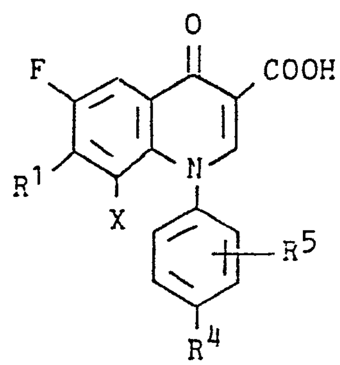

wherein $R^1$, $R^4$, $R^5$, and X are as defined above, or

(b) reacting a compound of the formula:

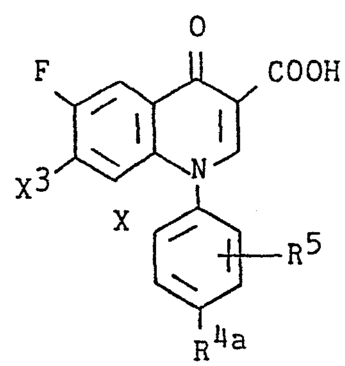

wherein $R^5$, $R^{4a}$ and X are as defined above, and $X^3$ is a halogen atom, with a compound of the formula: H-$R^1$ (wherein $R^1$ is as defined above), optionally followed by subjecting to conversion of alkoxy in $R^{4a}$ into hydroxy and further optionally to alkanoylation of hydroxy, to obtain a compound of the formula:

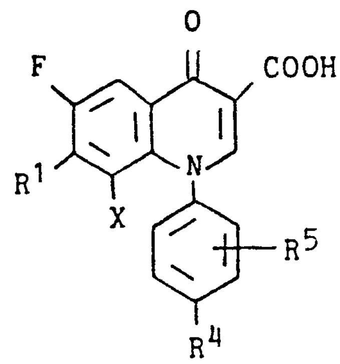

wherein $R^1$, $R^4$, $R^5$ and X are as defined above, or

(c) reacting a compound of the formula:

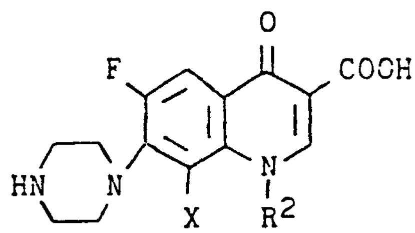

wherein $R^2$ and X are as defined above, with a compound of the formula: $R^{3'}X^5$ (wherein $R^{3'}$ is an alkyl having 1 tc 2 carbon atoms and $X^5$ is a halogen atom) to obtain a compound of the formula:

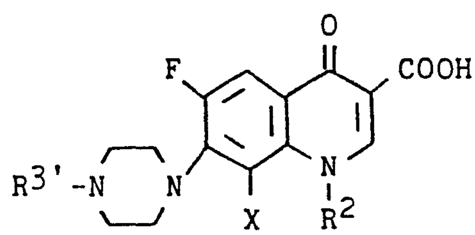

wherein wherein $R^2$, $R^{3'}$ and X are as defined above, or

(d) reacting a compound of the formula:

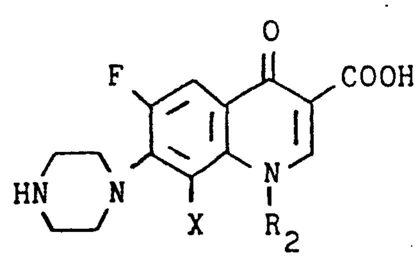

wherein $R^2$ and X are as defined above, with a compound of the

formula: $R^{17}CHO$ (wherein $R^{17}$ is hydrogen atom or methyl group) to obtain a compound of the formula:

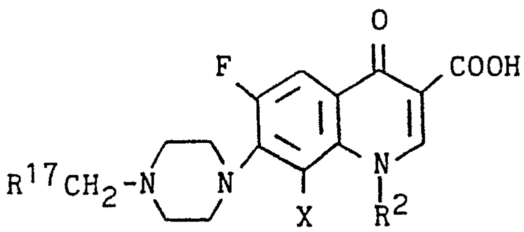

wherein $R^2$, $R^{17}$ and X are as defined above.

European Patent Office

# EUROPEAN SEARCH REPORT

0181521

Application number

EP 85 11 3203

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| Y | GB-A-1 147 336 (LILLY) * Claim 1; page 5, lines 113-117 * --- | 1,15 | C 07 D 215/56<br>C 07 D 487/04<br>A 61 K 31/495//<br>(C 07 D 487/04<br>C 07 D 241:00<br>C 07 D 209:00 ) |
| Y | US-A-4 398 029 (KYORIK) * Column 1, lines 16-56 * --- | 1,15 | |
| Y | FR-A-2 391 210 (KYORIN) * Claims 1,6 * --- | 1,15 | |
| Y | FR-A-2 424 919 (KYORIN) * Claims 1,5 * --- | 1,15 | |
| Y | FR-A-2 437 406 (KYORIN) * Claims 3-10 * --- | 1,15 | TECHNICAL FIELDS SEARCHED (Int. Cl 4) |
| P,X | EP-A-0 131 839 (ABBOTT) * Examples 4,5,12,13; claims 1,10 * --- | 1,15 | C 07 D 215/00<br>C 07 D 487/00<br>A 61 K 31/00 |
| P,X | EP-A-0 154 780 (ABBOTT) * Examples 15,16,18,19; claims 1,20 * ----- | 1,15 | |

The present search report has been drawn up for all claims

| Place of search | Date of completion of the search | Examiner |
|---|---|---|
| THE HAGUE | 15-01-1986 | ALFARO I. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82